(19) Europäisches Patentamt / European Patent Office / Office européen des brevets

(11) EP 4 122 459 A1

(12) EUROPEAN PATENT APPLICATION

(43) Date of publication:
**25.01.2023 Bulletin 2023/04**

(21) Application number: **22186396.2**

(22) Date of filing: **22.07.2022**

(51) International Patent Classification (IPC):
***A61K 31/16*** (2006.01) ***A61K 36/185*** (2006.01)
***A61K 36/28*** (2006.01) ***A61K 31/198*** (2006.01)
***A61K 31/593*** (2006.01) ***A61K 33/04*** (2006.01)
***A61K 33/30*** (2006.01) ***A61P 17/00*** (2006.01)
***A61K 8/23*** (2006.01) ***A61K 8/27*** (2006.01)
***A61K 8/44*** (2006.01) ***A61K 8/67*** (2006.01)
***A61K 8/9789*** (2017.01) ***A61P 17/06*** (2006.01)
***A61P 29/00*** (2006.01) ***A61P 31/04*** (2006.01)
***A61P 31/10*** (2006.01) ***A61Q 3/02*** (2006.01)

(52) Cooperative Patent Classification (CPC):
(C-Sets available)
**A61P 31/04; A61K 8/0229; A61K 8/44; A61K 8/9789; A61K 31/16; A61K 31/198; A61K 31/593; A61K 33/04; A61K 33/30; A61K 36/185; A61K 36/28; A61P 17/00; A61P 17/06; A61P 29/00; A61P 31/10;** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.07.2021 IT 202100019727**

(71) Applicant: **Giuliani S.p.A.**
**20129 Milano (IT)**

(72) Inventors:
- **GIULIANI, Giammaria**
  **Montagnola Switzerland (IT)**
- **RINALDI, Fabio**
  **20129 MILANO (IT)**
- **PINTO, Daniela**
  **20151 Milano (IT)**
- **MARZANI, Barbara**
  **27020 CARBONARA AL TICINO (IT)**

(74) Representative: **Coppo, Alessandro et al**
**Notarbartolo & Gervasi S.p.A.**
**Viale Achille Papa, 30**
**20149 Milano (IT)**

# (54) COMPOSITION FOR PREVENTING AND TREATING NAIL ALTERATIONS AND DISEASES

(57) The present invention concerns a composition for preventing and treating nail alterations and diseases comprising spilanthol, or a *Spilanthes acmella* extract which contains it, in combination with zinc and cocoa. The composition is formulated for topical or oral application. The composition accelerates the repair process of the structural alterations of the nail plate regardless of the traumatic, infectious, or nutritional origin.

EP 4 122 459 A1

FIG. 4

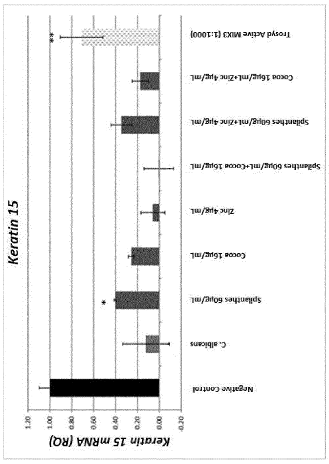

(52) Cooperative Patent Classification (CPC): (Cont.)
**A61Q 3/00**

C-Sets
**A61K 31/16, A61K 2300/00;**
**A61K 31/198, A61K 2300/00;**
**A61K 31/593, A61K 2300/00;**
**A61K 33/04, A61K 2300/00;**
**A61K 33/30, A61K 2300/00;**
**A61K 36/185, A61K 2300/00;**
**A61K 36/28, A61K 2300/00**

## Description

### Field of the Invention

The present invention concerns a combination composition for preventing and treating nail alterations and diseases.

The present invention originates in the pharmaceutical and nutraceutical sector.

In particular, the present invention relates to the use of a composition containing a combination of biologically active ingredients to restore physiological conditions of nail growth and malformations of its corneal structure or to prevent or treat nail diseases.

### State of the art

The nail is a semi-transparent and slightly convex quadrangular plate made of several layers of transparent and flattened keratinized keratinocytes free of keratohyalin granules and tonofibrils.

The nail originates from the nail matrix and grows along the nail bed, emerging through the hyponychium, commonly referred to as the cuticle. The hyponychium is located between the nail bed and the distal sulcus. The final portion of the nail detached from the nail bed is called the free edge, or distal edge.

In physiological conditions the nail growth is continuous at a rate of about 0.1-1 mm per day. The growth rate varies depending on some factors such as the age of the individual, health conditions, nutrition, and ambient temperature, during the summer months, in fact, it grows faster.

The nail mainly acts as an aid to gripping and to protect against external agents by constituting a semipermeable barrier which substantially prevents the penetration of pollutants and potentially harmful substances.

However, this protective function requires that the nail horny structure is intact and in good condition.

Some circumstances, such as deficiencies of suitable nutritional substances in the diet, trauma and diseases may contribute to determining conditions that favor the flaking of the horny structure and the development of nail diseases which in turn affect the functionality of the matrix up to a temporary stop of its activities.

In these conditions, morphological alterations of the nail horny structure occur which lead to breakage, flaking, peeling off, and the formation of furrows such as Beau's lines.

Damage to the nail structure can affect not only its growth, shape and size but also predispose the nail to infection and consequent inflammation due to soft tissue breakdown resulting in inflammation of the subcutaneous tissue.

It was also observed that the nail lamina, and the keratins which forms it, can be subject to oxidative stress that can determine a local inflammatory state.

Among causes that contribute to causing oxidative stress, there are exposure to UV rays, contact with aggressive chemicals such as organic solvents, glues and synthetic dyes. In these cases, the inflammatory state is accompanied by the release of reactive oxygen species (ROS) which favor the deterioration of the nail, making it even more susceptible to attack by pathogens, such as bacteria and fungi.

A further cause of inflammation is represented by trauma in the portions of the hands covered by the nails which cause a break in the horny structure thereof, significantly reducing the barrier effect against external agents, such as cosmetic products and pathogens.

In these cases, traditional local nail treatments are inadequate as they do not affect inflammatory processes and therefore do not solve one of the main causes of nail diseases.

Currently, there is a need to have products for the systemic treatment of nail diseases that act by reducing inflammation which is one of the main contributing causes of nail damage.

One of the objects of the invention is therefore to provide a composition containing a combination of active substances to improve the external appearance of the nail or to treat nail diseases.

Another object of the invention is to provide a composition or dietary supplement for the prevention and/or treatment of alterations in the horny structure of the nail or of nail diseases whose use is practically free of side effects.

### Summary of the invention

Within the technical sector of the invention, the Applicant has unexpectedly found that by combining selected biologically active components, a composition is obtained that accelerates the process of repairing the structural alterations of the nail plate regardless of the traumatic, infectious, or nutritional origin.

Furthermore, the inventors have observed how the combination of the selected biologically active components prevent and reduce the cellular oxidative stress of the nail matrix and surrounding areas, thus favoring the recovery of physiological conditions of nail growth.

These technical effects found by the Applicant are reflected in the experimental tests reported below, which

also highlight the synergistic activity of the combination of the base components of the composition of the invention.

In view of the aforementioned objects and of the experimental findings obtained, the present invention provides a composition comprising a combination of spilanthol, or a *Spilanthes acmella* extract which contains it, with zinc or a salt thereof, preferably bisglycinate zinc, and cocoa.

In particular, the *in vitro* tests in a model of co-infection with *C. albicans* of HACAT keratinocytes, reported herein and illustrated in the Figures, show that the composition described herein inhibits C. *albicans* growth, reduces β-tubulin and TNF-α, stimulates E-cadherin and Keratin 15 (K15).

Furthermore, the experimental evidence of the Examples shows how the composition described herein has an inhibiting activity against the biofilm, also referred to below as an anti-biofilm activity, which manifests itself as a reduction of the adhesion on the nail keratin component and inhibition of the biofilm development.

In particular, the *in vitro* tests in the HACAT keratinocyte co-infection model with *Staphylococcus aureus* show that the composition described herein inhibits S. *aureus* growth, reduces the formation of TNF-α inflammatory cytokines, stimulates cytokeratin 15 (K15) which in case of infection undergoes ruptures and degradation.

The composition may be for oral or topical use on the nails and surrounding anatomical areas.

According to some embodiments, the composition may further comprise a biologically active ingredient selected from selenium, valine optionally in the form of a salt or an ester, vitamin D preferably vitamin D3, ornithine optionally in the form of a salt or an ester, and mixtures thereof.

In one aspect, the invention provides the non-therapeutic use of a composition as described herein for improving the aesthetic appearance of the nails and/or for treating fractures, flaking, cracks, dulling of the nail and/or for accelerating nail regrowth.

Within the context of applications in the cosmetic or aesthetic field, the composition of the invention is specifically indicated in preventing or treating nail blemishes, in particular fractures, flaking, cracks, dulling, and in promoting the physiological nail growth.

These cosmetic effects of the composition are mainly attributable to the stimulation of cell proliferation and keratin 15, as highlighted in the attached Figures and experimentally demonstrated in the Examples.

In another aspect, the present invention relates to the composition as described herein for medical use.

In the medical field, the composition is suitable for the treatment or prevention of nail diseases of traumatic, infectious and/or inflammatory origin.

In particular, a further object of the invention is a composition as described herein for oral or topical use in the prevention or treatment of a nail infection of bacterial origin, in particular from S. *aureus,* or fungal in particular from *C. albicans.*

These therapeutic effects of the composition are mainly attributable to the antibiofilm activity, the inhibition of inflammatory processes associated with pathogen infections, and the inhibition of the lamina degradation processes associated with the infection by pathogens, as highlighted in the attached Figures and experimentally demonstrated in the Examples.

In another aspect, a composition is provided for oral or topical use in the prevention or treatment of nail trauma or dystrophy.

In a further aspect, the invention relates to a composition as described herein for oral or topical use in the prevention or treatment of inflammatory or psoriatic forms.

**Brief description of the drawings**

The characteristics and advantages of the present invention will become more evident from the accompanying drawings in which:

Fig. 1 depicts a representation of C. *albicans* biofilm stages

Fig. 2 illustrates bar graphs relating to TNF-α gene expression in HACAT cells co-infected with C. *albicans* and treated with the composition containing the three active components, Spilanthes extract, cocoa, and zinc, and with the individual components.

Fig. 3 illustrates bar graphs relating to *E-cadherin* gene expression in HACAT cells, as assessed by qRT-PCR. The cells were co-infected with C. *albicans* and treated with the composition containing the three active components, Spilanthes extract, cocoa, and zinc, and with the individual components.

Fig. 4 illustrates bar graphs relating to gene expression data of the *KRT15* (Keratin 15) marker after 24 hours of incubation with the compounds under analysis and C. *albicans.*

The greatest and significant increase in *KRT15* gene expression is evident in the presence of treatment with the three-component combination (1:1000). The effect is found to be synergistic with respect to the other combinations tested.

Fig. 5 illustrates bar graphs relating to the gene expression data of the Beta-tubulin marker after 24 hours of incubation

with the test compounds and C. *albicans.* The graphs show how the greatest and significant reduction in *Beta-tubulin* gene expression is attributable to the treatment with the three-component combination of the invention (1:1000) and this effect is found to be synergistic with respect to the other combinations tested.

Fig. 6 illustrates curves relating to C. *albicans* growth in the presence of the individual active components and of the combination thereof. The greatest reduction in the area covered by *C. albicans* is evident in the presence of the treatment with the combination of the invention.

Fig. 7 illustrates bar graphs of keratin 15, *KRT15,* gene expression data in HACAT cells. The greatest reduction in *KRT15* gene expression is evident in the presence of treatment with the three-component composition.

Figs. 8-9 illustrate bar graphs relating to proliferation data at 24 and 48 hours, respectively, of Hacat cells treated with Spilanthes (60ng/mL; 6ng/mL; 6μg/mL; 60μg/mL); Cocoa (16ng/mL; 160ng/mL; 1.6μg/mL;16μg/mL) and Zinc (4ng/mL; 40ng/mL; 400ng/mL; 4μg/mL). The extracts were treated in pairs and triplets in a 15:4:1 ratio at all tested concentrations. Untreated cells represent the negative control.

Figure 10 illustrates bar graphs showing S. *aureus* biofilm adhesion data under co-treatment with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL). The decrease in cell viability corresponds to a greater activity in contrasting the adhesion of the biofilm by S. *aureus.*

Figure 11 illustrates bar graphs of C. *albicans* biofilm adhesion data (expressed as % viability *versus* untreated control) under co-treatment with Spilanthes (60ng/mL; 6ng/mL; 6μg/mL; 60μg/mL); Cocoa (16ng/mL; 160ng/mL; 16μg/mL; ,16μg/mL) and Zinc (4ng/mL; 40ng/mL; 400ng/mL; 4μg/mL).The decrease in cell viability is representative of a greater activity in contrasting the development of the biofilm by *C. albicans.*

Figure 12 illustrates bar graphs of C. *albicans* biofilm development data expressed as % viability *versus* untreated control) under co-treatment with Spilanthes (60ng/mL; 6ng/mL; 6μg/mL; 60μg/mL); Cocoa (16ng/mL; 160ng/mL; 1.6μg/mL16μg/mL) and Zinc (4ng/mL; 40ng/mL; 400ng/mL; 4μg/mL). The decrease in cell viability is representative of a greater activity in contrasting the development of the biofilm by *C. albicans.*

Figure 13 illustrates bar graphs of C. *albicans* mature biofilm development data under co-treatment with Spilanthes (60ng/mL; 6ng/mL; 6μg/mL; 60μg/mL); Cocoa (16ng/mL; 160ng/mL; 16μg/mL; 16μg/mL) and Zinc (4ng/mL; 40ng/mL; 400ng/mL; 4μg/mL).A decrease in cell viability corresponds to a greater activity in contrasting the development of the biofilm by *C. albicans.*

## Detailed description of the invention

The present invention originates from having found that by combining spilanthol, preferably extracted from the plant *Spilanthes acmella,* with zinc and cocoa, certain specific effects are obtained on the nail, such as an acceleration of growth and repair of damage to the keratin structure and a reduction in adhesion and formation and development of bacteria and fungi biofilms.

These effects contribute to inhibiting the development of bacterial and fungal colonies on the nail by preventing and treating infections from pathogen agents and the related local inflammation with a reduction in the frequency of flaking, breakages, and cracks in the horny layer of the nail.

In addition, the combination of the biologically active components accelerates the nail keratinization process, improving the external appearance thereof, typically making them smoother to the touch, brighter and more compact, thus reducing the fragility thereof.

Consequently, the composition of the invention is also indicated in the non-therapeutic field in improving the external appearance of a subject's nails. According to a first aspect of the invention, a composition is therefore provided as defined in the attached claims 1-4.

Another object of the present invention is a food supplement for oral administration as defined in claim 6.

A further object of the invention relates to the cosmetic or aesthetic use of a composition as defined in the attached claim 6.

According to another aspect of the invention, a cosmetic treatment method is provided comprising the application on a nail of an effective amount of a composition for topical use as previously described. In applications in the cosmetic field, for example in improving the nail external appearance, it is possible to apply a cosmetically active amount of the composition of the invention on the affected skin area one or more times a day, conveniently for a period of at least 2-3 months.

According to another aspect, a composition is provided for use according to any one of claims 7-9.

Specifically, the composition described herein is for use in the treatment of nail diseases that include an alteration of the lamina, such as infectious forms of bacterial, fungal, traumatic/dystrophic, inflammatory origins, including for example the inflammatory or dystrophic forms due to psoriasis.

The combination of the selected biologically active components affects the main processes involved in pathological forms such as inflammatory processes, oxidation and deterioration of the nail plate, infection due to both bacterial

and fungal species.

Advantageously, the composition contains a combination of a *Spilanthes acmella* extract, typically titrated in spilanthol, with cocoa typically titrated in theobromine, preferably as a powder extract, and zinc.

Preferably the *Spilanthes acmella* extract, cocoa and zinc components are present in the composition in 10-20:0.5-1.5:2-6 ratios, more preferably in a 15:1:4 ratio.

According to certain embodiments, the composition contains at least one of the following further biologically active components: selenium in free form or in a product containing it, valine optionally in the form of a salt or ester, vitamin D, preferably D3, ornithine optionally in the form of a salt or ester.

In a preferred embodiment, the composition contains the amino acid valine optionally in the form of a salt or ester. The combination of valine with the other three components of the composition carries out a structural function and helps to restore the nail plate and accelerates the natural processes of formation of the keratins that make it up. Its combination with the other three components increases the antioxidant action by reducing the oxidative stress of the nail structures, for example resulting from irradiation, the application of chemicals, trauma. It has in fact been observed that the release of reactive oxygen species (ROS) favors the deterioration of the nail, making it even more susceptible to aggression from physical and pathogenic agents. The combination of the biological components according to an embodiment described herein counteracts the oxidative stress also at the nail plate level.

The presence of zinc and cocoa theobromine, and optionally selenium, counteracts the action of free radicals and exerts an anti-inflammatory action thanks to the ability to downregulate pro-inflammatory cytokines.

According to preferred embodiments, the composition further contains ornithine, an amino acid derivative which, when combined with the three components of the composition and preferably also with valine, increases the biosynthesis of spermidine, a polyamine that stimulates nail growth and regeneration.

Damage to the nail structure can affect not only its growth, shape and size but also predispose the nail to infection and consequent inflammation due to soft tissue breakdown resulting in inflammation of the subcutaneous tissue.

The nail repairing action of the composition according to an embodiment described herein prevents the infiltration of pathogenic microorganisms in the underlying tissues, thus reducing the incidence of infections.

In view of these activities, the composition of the invention is suitable for both medical and non-therapeutic uses, in the treatment of nails.

The biologically active components present in the composition are described in detail below.

One of the biologically active components present in the composition, Spilanthol, may be of natural or synthetic origin.

In the composition of the invention, Spilanthol or a portion of a *Spilanthes acmella* plant, or preferably an extract thereof containing spilanthol, may be present. *Spilanthes acmella* is a species belonging to the Spilanthes genus of the Compositae or Asteraceae family.

The plant or a portion thereof may be used as a whole, ground, or preferably as an extract. Parts of the plant include leaves, seeds, inflorescences, roots, or the stem/shaft. As part of the invention, the use of an extract from the plant inflorescences or roots is preferred.

The main chemical compounds isolated and contained in a plant extract of *Spilanthes acmella* include alkylamides or alkamides, preferably spilanthol or affinin (2E,6Z,8E)-N-isobutyl-2,6,8-decatrienamide (Gokhale and Bhide, 1945[31]; Ramsewak *et al.,* 1999[76]) identified with the number 1 and/or amide derivatives 2-9 as illustrated below.

Among these compounds, for the uses of the invention spilanthol is preferred, but mixtures of the nine compounds may also be used.

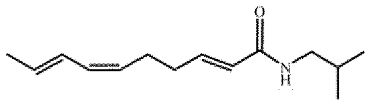

1

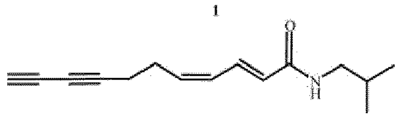

2

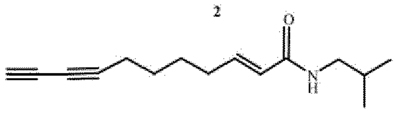

3

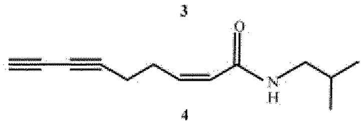

4

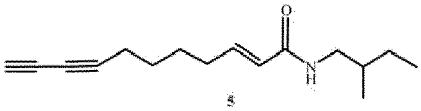

5

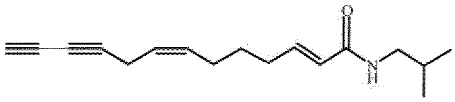

6

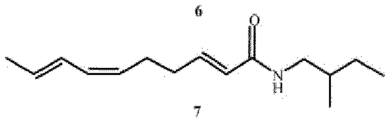

7

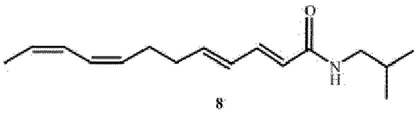

8

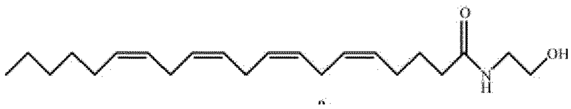

9

It is possible to obtain a vegetable extract from *Spilanthes acmella* using conventional extraction techniques, for example by maceration or solid-liquid techniques suitable for separating/extracting one or more biologically active components from the vegetable tissues of plants and fruits thereof.

A suitable solvent to obtain the *Spilanthes acmella* plant extract is a physiologically acceptable liquid in which the biologically active components are soluble and in which they do not undergo any alterations depriving them of activity.

Suitable solvents are non-polar solvents, such as hexane (Ramsewak *et al.,* 1999) or protic polar solvents, preferably water, methanol, ethanol, and mixtures thereof, such as ethanol or methanol: $H_2O$, for example 4:1, v/v.

A preferred physiologically acceptable solvent is a hydroalcoholic mixture, preferably water-ethanol. Hydroalcoholic extraction is particularly suitable when using inflorescence as the starting material for the extraction.

By way of example, the hydroalcoholic solvent may contain from 10% to 70% by volume of alcohol, preferably ethanol, preferably from 20% to 60% by volume, still preferably from 30% to 50% by volume, for example 40% by volume.

The extraction may be cold or hot extraction, for example at 40-70 degrees centigrade.

The solvent extraction of the biologically active components present in the plant tissues can thus take place by diffusion and osmosis

In certain embodiments, the extraction is carried out using a weight ratio between solvent and plant matrix comprised between 1:10 and 10:1.

A suitable extract from *Spilanthes acmella* can be fluid, soft or dry.

For example, in the fluid extract, 1 mL of extract contains the biologically active components soluble in 1 g of vegetable drug; in the soft extract the solvent is partially evaporated, in particular until the extract wets a filter paper; in the dry extract the solvent is evaporated almost completely to obtain a powder.

In the formulation of the composition, a dry extract is preferably used, advantageously from the root or inflorescence of *Spilanthes acmella.*

For example, an initial stage of the preparation of a suitable dry extract provides for the plant or portions thereof, preferably inflorescences or roots, to be cleaned, dried, and optionally cut into pieces or chopped, ground, or pulverized.

At a later stage this material is extracted using conventional methods.

A suitable extraction provides for a portion or plant matrix, preferably inflorescence of *Spilanthes acmella,* to be ground to give a powder from which the extraction is carried out using a suitable solvent, such as a mixture of water-ethanol, or ethanol, for example from 40 to 80% in ethanol, for a time suitable for enriching the solvent with one or more biologically active components, for example 10 minutes.

In certain embodiments the maceration time may vary between 10 minutes and 48 hours, 1 and 5 hours.

Under these conditions, the extraction of the biologically active components present in the plant tissues by the solvent occurs by diffusion and osmosis.

In certain embodiments, the extract obtained may be sonicated and centrifuged, for example at 3000 rpm for 4-6 minutes. The extract obtained is collected and may be diluted with an alcohol, typically ethanol, for example to obtain a final volume of 10 mL when starting from 1 gram of initial powder.

The extracted liquid may be filtered, if necessary. At a later stage, the liquid extract is dried to obtain the biologically active component in the form of a dry powder. Drying may be achieved with conventional techniques such as air, heating, or spray-drying.

Additional methods for obtaining the *Spilanthes acmella* plant extract, titrated in Spilanthol, include extraction techniques by digestion, infusion, squeezing, decoction, percolation, counter-current extraction, Soxhlet, extraction with supercritical gases such as $CO_2$, or ultrasounds.

According to some embodiments, the *Spilanthes acmella* dry extract is a dry extract from the whole plant, or preferably from roots, titrated in spilanthol.

For example, a dry extract from *Spilanthes acmella* contains from 0.5% to 5%, from 1 to 3% of spilanthol, which is the main biologically active ingredient contained in the plant.

Advantageously, a dry inflorescence extract of *Spilanthes acmella* titrated at 3% in spilanthol can be used in the composition.

In certain embodiments, the *Spilanthes acmella* plant extract is present in the composition in an amount from 0.01 to 20%, from 0.5 to 20%, from 1 to 10%, for example 5% by weight with respect to the total weight of the composition.

In certain embodiments, the composition of the invention contains a *Spilanthes acmella* dry extract in an amount ranging from 5 to 2000mg, from 100 to 1200, from 200 to 600mg, for example 400 mg per dosage unit.

Another selected component of the composition is preferably cocoa powder obtained from the *Theobroma cacao* plant, belonging to the family of *Sterculiaceae* native to South America.

Advantageously, cocoa bean powder from *Theobroma cacao* is present in the composition.

A suitable cocoa powder for the formulation of the composition described herein may be obtained with a process including the following steps:

a) providing fermented or unfermented cocoa beans;
b) draining/dripping the beans from step a)
c) drying the drained beans;
d) heating the cocoa beans at a temperature from 70 to 120°C for a period of time suitable to remove the shell of the beans, for example for 1 - 30 minutes, for example with infrared or hot air, for example at 30-70°C, 45-60°C.
e) sieving/separating the heated beans to separate cocoa grains/beans/seeds from the shell;
f) grinding cocoa beans/grains/seeds into a dark brown fluid broth containing the ground particles of partially lipid-free (partially delipidated) cocoa solids suspended in cocoa butter;
g) separating the cocoa solids from the cocoa butter.

According to some embodiments, the amount of cocoa present in the composition of the invention ranges from 10 to 1000 mg, preferably from 50 to 500 mg, more preferably from 100 to 300 mg, for example 200 mg per dosage unit.

According to some embodiments, the amount of cocoa, preferably in powder form, ranges from 10 to 1000 mg, preferably from 50 to 500 mg, more preferably from 100 to 300 mg, for example 200 mg per dosage unit.

In particular, the cocoa bioactive components reduce inflammatory processes. This activity is based on their ability to downregulate pro-inflammatory cytokines and their downstream biochemical pathways.

Among the components present in cocoa from *Theobroma cacao* there is Theobromine, a xanthine belonging to the chemical group of purine bases capable of inhibiting phosphodiesterase, an enzyme that catalyzes the conversion of cAMP into 5 AMP (inactive). Therefore, the intracellular accumulation of cAMP follows, not due an increased production thereof, but to an extension of the half-life by inhibition of its degradation.

These premises have directed several authors towards the topical use of xanthines in the androgenetic course at concentrations ranging from 0.2 to 2% in an attempt to lengthen the anagen phase. Numerous *in vivo* and *in vitro* studies have highlighted the transcutaneous penetration properties of xanthines (Bronaugh R.L. - Feldmann R.J. - Rogers J.G. - Zesch A.). The results of this type of therapeutic approach, monitored by trichogram, were considered interesting (Seiler W.G.) and therefore, also considering their absolute harmlessness, it is considered that xanthines could be used

as routine topical drugs in the early treatment of common baldness. Xanthines act by activating the protein kinase system and modulating the energy availability for the protein synthesis of the hair. In certain embodiments the cocoa extract, preferably a dry extract, contained in the composition in an amount from 0.01 to 20%, from 0.5 to 20%, from 0.5 to 8%, for example 2% by weight with respect to the total weight of the composition.

In certain embodiments, the composition of the invention contains a cocoa extract, preferably dry extract, in an amount ranging from 1 to 1000 mg, from 5 to 500 mg, from 20 to 200 mg, for example 50 mg.

A further biologically active component of the composition described herein is zinc, a mineral or trace element that plays an important role in cellular metabolism and is involved in important biological functions, including DNA synthesis, gene expression, hormonal control, enzymatic reactions, and cell proliferation.

Zinc is involved as a structural element and/or regulatory factor and plays a role in important functional activities within the hair follicle. It is essential in the synthesis of proteins and also plays a very important role in the development of nails and hair.

In addition, zinc is a 5 alpha-reductase inhibitor and therefore contributes to blocking DHT. As a cofactor, it is involved in 60 enzymatic reactions; for example, the superoxide-dismutase enzyme, an antioxidant that counteracts the action of free radicals, can have different metals as cofactors, including zinc.

Furthermore, zinc is a constituent of the nail plate, and its nutritional deficiency may affect nail growth and cause diseases.

In the composition or supplement, zinc may be present in elemental form, or as an oxide or salt, for example zinc chloride, zinc acetate, zinc citrate, or zinc picolinate, or bisglycinate zinc, for example at 20% zinc.

According to some embodiments, the composition of the invention contains zinc, preferably zinc bisglycinate, in an amount ranging from 0.01 to 10% by weight, from 0.1 to 10% by weight, from 0.5 to 5%, for example 1% by weight.

For example, the composition or supplement described herein may contain from 0.1 to 100 mg, from 1 to 50 mg, from 2 to 20 mg of zinc or zinc bisglycinate per dosage unit.

Another vitamin component of the composition is vitamin D. This vitamin, also known as calciferol, comprises a group of fat-soluble secosteroids that exist in different forms, the most widespread and of greatest biological interest of which are vitamin D2, known as ergocalciferol, and preferably vitamin D3, known as cholecalciferol.

Vitamin D increases the absorption of calcium, an essential mineral for the structure of bones and nails, which contributes to the normal absorption of calcium and phosphorus and to the maintenance of physiological levels of calcium in the blood.

Furthermore, the presence of vitamin D3 in the composition increases the absorption of calcium, an important mineral for the structure of the nails, and also contributes to the normal immune system functions, by exerting an immunomodulatory/immunostimulating activity, useful for the defense from external aggressions and infectious agents.

According to some embodiments, the composition of the invention contains vitamin D, preferably D3, in an amount ranging from 0.01 to 10% by weight, from 0.1 to 10% by weight, from 0.5 to 5%, for example 1% by weight.

For example, the composition or supplement described herein may contain from 0.1 to 100 mg, from 1 to 50 mg, from 2 to 20 mg of vitamin D, in particular D3, per dosage unit.

The composition may further contain one or more amino acids, preferably valine. This amino acid, together with Leucine and Isoleucine, is part of the branched amino acids group and, as such, is part of the energy metabolism. It is an essential amino acid and, as such, it is indispensable for the synthesis of proteins including the keratin of nails and hair, in which it is present in significant amounts (about 6%).

According to some embodiments, the composition of the invention optionally contains valine as a salt or ester, in an amount ranging from 0.01 to 10% by weight, from 0.1 to 10% by weight, from 0.5 to 5%, for example 1% by weight.

For example, the composition or supplement described herein may contain from 1 to 1000 mg, from 20 to 300 mg, from 35 to 150 mg of valine or salt or ester, per dosage unit.

Another suitable micronutrient that may be present in the composition is selenium, a mineral present in trace amounts in the human body, provided with anti-inflammatory and antioxidant activity. Selenium may be present in the composition in elemental form. Alternatively, it may be present in the form of selenium containing yeast.

Selenium is the cofactor of several selenoproteins whose functions play an essential role in maintaining hair and nails in physiological conditions.

Serum selenium is present in the body:

about 60% in Selenoprotein P (SePP);
30% bound to Glutathione peroxidase (GPx);
From 5% to 10% bound to serum albumin;
Less than 1% in free form.

More than 50% of selenoproteins are endowed with antioxidant activity. Among these, the best known are the GPx and Thioreduxin reductase families (Trx R). GPx comprise eight isoforms, which catalyze the reduction of various

hydroperoxides, acting synergistically with α-Tocopherol in the antioxidant defense against lipid peroxidation.

Selenoproteins may play a protective role against endothelium oxidative damage. They inhibit the activation, through the redox signal, of the NF-κB factor and thus stop the cytokine storm as well as the formation of reactive oxygen and nitrogen species.

According to some embodiments, the composition of the invention contains selenium or selenium-based compounds, such as selenium-L-methionine, in an amount ranging from 0.0001 to 1% by weight, from 0.001 to 0.1% by weight, from 0.005 to 0.05%, for example 0.01% by weight.

For example, the composition or supplement described herein may contain from 1 to 1000 mcg, from 5 to 500 mcg, from 20 to 200 mcg of selenium, for example 60 mcg per dosage unit.

According to some embodiments, the composition contains ornithine or a salt or ester thereof, an amino acid derivative that originates from the activity of the arginase enzyme that cleaves L-arginine into ornithine and urea. Preferably ornithine is in the form of L-ornithine hydrochloride.

In mammalian non-hepatic tissues, the primary metabolic role of the urea cycle is the synthesis of arginine, but also the biosynthesis of L-proline and polyamines, including spermidine.

Ornithine plays an important role as an intermediate product of metabolic processes. It has similar effects to arginine, but with greater efficacy.

In certain embodiments, ornithine, preferably ornithine hydrochloride, is present in the composition in an amount from 0.01 to 20%, from 0.5 to 20%, from 0.5 to 8%, for example 2% by weight with respect to weight total composition.

In certain embodiments, the composition of the invention contains ornithine, preferably ornithine hydrochloride, in an amount ranging from 1 to 1000 mg, from 5 to 500 mg, from 20 to 200 mg, for example 50 mg.

In one embodiment, the composition contains all six biologically active ingredients previously described.

The composition of the invention may be intended for both external use and oral administration.

The composition of the invention may be authorized on the market as a cosmetic product for local application, or as a drug, dietary or nutritional supplement intended for oral administration.

Typically, the composition of the invention comprises a physiologically and/or cosmetically acceptable carrier, diluent, or excipient.

Typically, the physiologically or cosmetically acceptable carrier of the composition of the invention is an excipient, carrier, or diluent suitable for topical application and/or for oral administration.

In the present context, the term "carrier" refers to one or more of an excipient, carrier, diluent, or adjuvant that may be present in the composition of the invention. Any carrier and/or excipient suitable for the form of preparation desired for administration is contemplated in the uses of the plant extract or active ingredients present therein described herein.

In certain embodiments, the composition of the invention contains one or more components of plant origin which are biologically active and substantially free of side effects, when administered orally or locally.

The pharmaceutically and/or physiologically acceptable carrier, diluent, or excipient may be selected based on the route of administration for which the resulting pharmaceutical composition is intended.

According to some preferred embodiments, the composition may be in a form for oral administration.

The compositions for oral administration may be in solid or liquid form.

Solid form compositions include tablets, capsules, powders, granules, pills.

Liquid form compositions include solutions, emulsions, suspensions, syrups. All compositions also include controlled release forms of the same.

A preferred embodiment of the composition is that in granules which can be dissolved in water at the time of administration.

The granules comprise a suitable carrier or excipient in which the components of the composition are dispersed.

Suitable excipients contained in the formulation are cellulose and derivatives thereof, such as hydroxymethylcellulose, hydroxypropylmethylcellulose, methylcellulose, hydroxypropylcellulose, hydroxyethylcellulose, carboxyethylcellulose, ethylhydroxyethylcellulose, carboxymethylcellulose, cellulose acetate butyrate, cellulose acetate phthalate, and mixtures thereof. Further examples of suitable excipients include polymers belonging to the lactam family, such as pyrrolidone and derivatives thereof, for example polyvinylpyrrolidone, polyvinylpolypyrrolidone and mixtures thereof, inorganic salts such as calcium or di-calcium phosphate, lubricants such as magnesium stearate, triacylglycerols, and mixtures thereof.

The composition may further comprise one or more of the following excipients: bulking agents such as calcium phosphates, stabilizers such as cross-linked sodium carboxymethylcellulose, coating agents such as polyvinyl alcohol, polyethylene glycol, talc, anti-caking agents such as magnesium salts of fatty acids; silicon dioxide.

The biologically active synergistic components contained in the composition of the invention may be present in variable amounts, for example ranging from 0.0001% by weight to 50% by weight, from 0.1% by weight to 20% by weight, typically from 0.5 to 5% by weight.

According to certain embodiments, the composition of the invention further comprises one or more active

substances such as vitamins, minerals, micronutrients, and other active substances.

According to some embodiments, the composition for oral administration is a food supplement, a functional food, a nutraceutical composition, a dietary product, a complement, or nutritional product, or a medical device.

Functional food means any modified food or food ingredient that, in addition to the traditional nutrients it contains, can provide a benefit or protection against a physiological disadvantage or disease.

Nutraceutical product means a product isolated or purified from edible substances. A nutraceutical is such when it is shown that it has a physiological benefit or that it provides protection against a physiological inconvenience or disorder.

Food or dietary supplement means a product that contains a vitamin, mineral, plant extract, amino acid, metabolite, extract, concentrate or mixture of these ingredients.

The amount administered and the frequency of administration of the composition will depend on the nature and severity of the condition to be treated.

In some embodiments, the route of administration of the composition of the invention is the topical route. According to these embodiments the composition is for topical use and is applied to the skin.

The composition for topical application may be in solid, semi-solid or fluid form. Suitable solid formulations include creams, pomades, pastes, ointments.

In other embodiments, the formulation for local administration is in fluid form, for example in the form of lotions, suspensions, or semi-fluid, for example in the form of gels and emulsions.

In some embodiments the compositions of the invention may comprise excipients commonly used in the formulation of cosmetic or pharmaceutical preparations, for local use, such as preservatives, bactericidal agents, stabilizers, emulsifiers, buffers, humectants, dyes, and other excipients commonly used in the techniques of cosmetic / pharmaceutical preparation.

The compositions may conveniently be presented in a unit dosage form and prepared according to any of the methods well known in the art of processing food, dietary products.

In some embodiments, such compositions and preparations may contain at least 0.1% of each of the biologically active substances.

Several other materials may be present as coatings or materials suitable for modifying the physical form of the dosing unit.

According to some embodiments, in the composition of the invention, the combination of active ingredients is formulated in dosage units, for example granules packaged in sachets. The dosage unit may contain each of the active ingredients in the amounts described above per dosage unit.

The present invention will now be described with reference to the following examples which are provided for illustrative purposes only and are not to be construed as limiting the present invention.

## EXAMPLES

### EXAMPLE No.1

Composition in the form of granules in a single dosage unit, preferably a sachet or stick, containing the following active substances

| | |
|---|---|
| 20% Zinc Bisglycinate | 7.50 mg/stick |
| Selenium yeast (lalminSe2000 | 0.060mg/stick |
| Vitamin D3 in powder | 0.015 mg/stick |
| L-Ornithine monohydrochloride | 50.00 mg/stick |
| *Theobroma cocoa* d.e. (% theobromine) | 50.00 mg/stick |
| Valine | 75 mg/stick |
| *Spilanthes acmella* | 400 mg/stick |

### EXAMPLE No.2

Food supplement to improve the aesthetic appearance of the nails, or to treat infections or inflammatory forms or dystrophies, having the following formulation:

| | | |
|---|---|---|
| *Spilanthes acmella* Extract 5:1 | *Spilanthes acmella* (L.) L. aerial part with flowers dry extract, Maltodextrins | 15-16 |

(continued)

| | | |
|---|---|---|
| Vitamin D3 100 CWS/AM | Gum arabic (acacia gum), Sucrose, Cornstarch, Medium chain triglycerides, Cholecalciferol (Vitamin D3), DL-alpha tocopherol | 0.2-0.3 |
| 6% Cocoa Extract | Cocoa (Theobroma cocoa L.) seeds dry extract, Corn maltodextrins | 4-5 |
| Ornithine-L hydrochloride (CN) | L-Ornithine hydrochloride | 2-3 |
| 28.2% Zinc bisglycinate Food grade Cod. 833500 | Zinc bisglycinate | 1-2 |
| L-Valine | L-Valine | 3-4 |
| Selenium yeast 2000 mcg/g Cod. 4591 | Selenium enriched yeast | 1-1.5 |
| Glucidex IT19 | Corn maltodextrins | 53-54 |
| Syloid 244 FP Cod. 7890 | Silicon dioxide | 0.5-1 |
| Chocolate Aroma SD | Aroma | 11-12 |
| Sucrasweet | Sucralose | 0.1-0.2 |
| E950 Acesulfame K Vitasweet | Acesulfame K | 0.6-0.7 |
| Sodium cyclamate | Sodium Cyclamate | 0.08-0.09 |
| Burnt sugar P-WS E6003 - 1301887 | Aroma | 5-6 |
| Methocel E5 Premium LV | Hydroxy-propyl-methylcellulose | 0.2-0.3 |
| | | 100 |

EXAMPLE No. 3

Composition for topical use in the form of a cream to be applied on the nails and in the surrounding area having the following formulation:

| **Component** | **INCI** | **Range %** |
|---|---|---|
| Purified water | AQUA | 16-17 |
| Denatured ethyl alcohol, type C | ALCOHOL DENAT. | 70-75 |
| 70% Glycolic acid | GLYCOLIC ACID, AQUA | 2.5-3 |
| Eudragit RL100 | ACRYLATES/AMMONIUM METHACRYLATE COPOLYMER | 10-10.5 |
| 6%Cocoa Extract | Cocoa *(Theobroma cocoa L.)* seeds dry extract, CORN MALTODEXTRINS | 0.5-0.6 |
| *Spilanthes acmella* Extract 5:1 | *Spilanthes acmella* (L.) L. aerial part with flowers dry extract, MALTODEXTRINS | 0.10-0.15 |
| 28.2% Zinc bisglycinate Food grade Cod. 833500 | Zinc bisglycinate | 0.03-0.04 |

EXAMPLE No. 4

1. OBJECT

Evaluation of the efficacy of the mixture/combination of Spilanthes, Zinc and Cocoa in the applications referred to in Paragraph 2 below. In the tests, the activities of the three individual components of the composition and of the combination thereof in the composition of the invention were compared to assess the confirmation of the synergistic effect.

2. TESTED USES

Evaluation of cosmetic use in the cosmetic treatment of nail changes due to trauma and dystrophies.

Evaluation of medical use in the treatment of nail alterations due to bacterial and/or fungal infectious forms.

For this purpose, microbiological *in vitro* tests and *in vitro* tests with cell lines (HACAT cells) were used

3. MATERIALS

.3.1 Tested samples

| INTERNAL NAME | *SPILANTHES ACMELLA* | Zinc | Cocoa extract (6% theobromine) |
|---|---|---|---|
| LAB ID: | S | C | Z |
| LOT: | ND | 2020041769 | 2020073577 |
| STORAGE: | r.t. | r.t. | r.t. |
| CONCENTRATION: | 60ng/m L- 600ng/mL- 6ug/m L- 60ug/m L | 4ng/m L- 40ng/m L- 400ng/mL- 4ug/m L | 16ng/mL- 160ng/mL- 1,6ug/mL- 16ug/mL |

All extracts were diluted to a stock concentration equal to the concentration in the finished product in culture medium (Stock solution-solubility 100%) and sterile filtered. The stocks were stored at - 20°C.

The extracts were tested *in vitro* at a final concentration equal to a dilution from 1:1000 to 1:1000000 on the cell culture with respect to the concentration in the finished product.

3.2 Reagents and instrumentation used

| REAGENTS | SUPPLIER |
|---|---|
| Agarose (For routine use) | SIGMA, A9539-100G |
| RPMI-1640 MEDIUM | SIGMA, R0883 |
| FETAL BOVINE SERUM | SIGMA, F7524 |
| Dimethylsulfoxide | SIGMA, D2438-50ML |
| Gentamicin solution | SIGMA, G1272 |
| L-glutamine | SIGMA, G7513 |
| Dulbecco's Phosphate Buffered Saline | SIGMA, D8537 |
| Ethidium bromide solution (10 mg/mL, for molecular biology, aqueous solution) | SIGMA, E1510 |
| Gel Loading Buffer RNAse, none detected | SIGMA, G2526 |
| Penicillin-Streptomycin | SIGMA, P0781 |
| PRIME SCRIPT RT reagent kit (Perfect Real time | Takara |
| PreMix Ex Taq | TAKARA, RR039A |
| TaqMan® Gene Expression Assays for GAPDH Hs99999905_m1 | APPLIED BIOSYSTEMS, ∠ |
| TaqMan® Gene Expression Assays for TNF-α Hs00174128_m1 | APPLIED BIOSYSTEMS, ∠ |
| TaqMan® Gene Expression Assays for KRT15 Hs00951967_g1 | APPLIED BIOSYSTEMS, 4331182 |
| TaqMan® Gene Expression Assays for TUBB2A Hs00742533_s1 | APPLIED BIOSYSTEMS, 4331182 |
| TaqMan® Gene Expression Assays for CDH1 Hs01023894_m1 | APPLIED BIOSYSTEMS, 4331182 |

(continued)

| REAGENTS | SUPPLIER |
|---|---|
| Trypsin-EDTA solution | SIGMA, T3924 |
| *Candida albicans* (Robin) Berkhout (3147) | ATCC- 10231 ™ |
| *Staphylococcus aureus subsp. aureus* | ATCC-25923™ |
| Sabouraud Glucose Broth Nutriselect plus | S3306-500g |
| MANNITOL SALT BROTH DISIDR 500gr | Biotech-6641 |

| INSTRUMENTATION | SUPPLIER |
|---|---|
| QiaExpert | Qiagen |
| 15 L digital water bath from +5°C to + 100°C (Mod: Swbd1, BS-SWB2D) | Stuart |
| Balance (Mod. XS204) | Mettler Toledo |
| Laminar flow cabinet (Mod: Gemini) + UV lamp with anti-reflex equipment | SterilManifacturingDivision |
| HeraCell $CO_2$ incubator (Mod: 150 ADV) | ThermoScientific |
| 85°C horizontal freezer ULT130, 120 L (Mod: Labfrost, MME-TE21140) | Elcold |
| Bürker counting chamber w/clamps (DI-DA-443/3) | Carlo Erba |
| Microplateautoreader (EL 808) | Biotek |
| Vortex | Arhos160-PBI International |
| MX3000p RT instrument | Stratagene |

3.3 Materials

### 3.3.1 HUMAN KERATINOCYTES CELL MODEL, HACAT CELLS

The immortalized line of human keratinocytes HACAT (BS CL 168) obtained from the Istituto Zooprofilattico (Brescia, Italy), kept in culture in sterile flasks (25 $cm^3$), incubated at 37°C in a humid atmosphere with 5% $CO_2$ in RPMI culture medium supplemented with 10% fetal bovine serum (FBS), in the presence of 1% penicillin and streptomycin, is used.

The 1:3 split is performed every 2 days upon reaching the monolayer by washing with 1X PBS (phosphate buffer without $Ca^{2+}$ and $Mg^{2+}$) and detaching the cells with a trypsin-EDTA solution at 37°C for 2 minutes. The cells were kept in culture in sterile 25 $cm^3$ flasks and incubated at 37°C in a humid atmosphere with 5% $CO_2$.

| ICLC CATALOG CODE | BS CL 168 |
|---|---|
| DEPOSITOR | Istituto Zooprofilattico (Brescia, Italy). |
| BIBLIOGRAPHIC REFERENCES | • Boukamp P., Dzarlieva-Petrussevska R.T., Breitkreutz D., H J., Markham A., Fusenig N.E.Normal keratinization in a spontaneously immortalized human keratinocyte cell line.J. 106:761-771 (1988)<br>• Schurer N., Kohne A., Schliep V., Barlag K., Goerz G.Lipid composition and synthesis of HaCaT cells, animmortalized keratinocyte line, in comparison with normal human adult keratinocytes.Exp. Dermatol. 2: 179-185(1993)<br>• Lehman R.T., Modali R., Boukamp P., Stanek J., Bennett W Welsh J.A., Metcalf R.A., Stampfer M.R., Fusenig N.E., Rog Harris C.C.p53 mutations in human immortalized epithelial ○ Carcinogenesis, 839(1993) |

### 3.3.2 MICROBIAL CULTURES

The C. *albicans* ATCC-10231™ strain (LGC Standard, Italy) was cultured in Sabouraud broth at 25°C and re-inoculated every 24h in fresh culture medium at 4%.
The S. *aureus* ATCC-25923™ strain (LGC Standard, Italy) was cultured in Mannitol salt broth at 37°C and re-inoculated every 24h in fresh culture medium at 4%.

### 3.3.3 CONTROLS

*3.3.2.1 In vitro test in a HACAT cell co-infection model with C. albicans (Kanchanapibon et al., 2020) or S. aureus (Gunarstam et al., 2019)*

NEGATIVE CONTROL: Untreated cells in RPMI added with 10% fetal bovine serum (FBS), in the presence of 1% penicillin and streptomycin and kept in 25 $cm^2$ culture plates (12 wells) at 37°C and 5% $CO_2$.
POSITIVE CONTROL: Cells pre-treated with *C. albicans* or S. *aureus* in RPMI added with 10% fetal bovine serum (FBS), in the presence of 1% penicillin and streptomycin and kept in 25 $cm^2$ culture plates (12 well) at 37°C and 5% $CO_2$.

*3.3.2.2 In vitro cell proliferation assay (MTT assay)*

NEGATIVE CONTROL: Untreated RPMI cells added with 10% fetal bovine serum (FBS), in the presence of 1% penicillin and streptomycin and kept in 25 $cm^2$ culture plates (12 well) at 37°C and 5% $CO_2$.

*3.3.2.3 Anti-biofilm activity*

NEGATIVE CONTROL: Untreated RPMI cells added with 10% fetal bovine serum (FBS), in the presence of 1% penicillin and streptomycin.
POSITIVE CONTROL: Cells treated with C. *albicans* or S. *aureus* in culture medium.

3.4 Methods

### 3.4.1 *In vitro* test in HACAT cell co-infection model with C. *albicans* (Kanchanapibon et *al.,* 2020) or S. *aureus* (Wiegand *et al.,* 2009)

The immortalized line of human keratinocytes HACAT (BS CL 168) obtained from the Istituto Zooprofilattico (Brescia, Italy), kept in culture in sterile flasks (25 $cm^3$), incubated at 37°C in humid atmosphere with 5% $CO_2$ in RPMI culture medium supplemented with 10% fetal bovine serum (FBS), in the presence of 1% penicillin and streptomycin, was used for the co-infection. Upon reaching confluence, the cells were seeded in 24-well plates at a cell density of $0.5*10^6$ cell/well and incubated for 24h at 37°C, with 5% $CO_2$.After 24h, fresh cultures (24h) of C. *albicans* and S. *aureus* were centrifuged (7500 rpm, 10 min), washed and resuspended in saline. Their cell density was calibrated at $1*10^6$ cells/well and $4.5*10^5$ cells/well for C. *albicans* and S. *aureus,* respectively, by measuring the optical density at 600 nm.
Then, 1mL of the respective cell suspensions were incubated alone (positive control) or with the active substances of interest according to the concentrations reported in Table 1. Spilanthes (S), Zinc (Z) and Cocoa (C) were tested individually, in pairs and triplets at all the concentrations indicated above for 24h. Uninfected cells were used as a negative control.
With regard to the co-infection with C. *albicans* at T0 after 120 min and after 24h treatment, microscope images (4x magnification) were also acquired in order to visually monitor the possible inhibition of colonization by *C. albicans* by the active substances tested.
In order to evaluate the protection from fungal or bacterial infection, the gene expression of the following markers was evaluated:

*C. albicans infection:*

- E-cadherin: adhesion protein whose degradation increases in the presence of C. *albicans* infection (Villar CC *et al.,* 2007)
- TNF-alpha (Lilic D *et al.* 2003)

- Cytokeratin 15 (in case of fungal or bacterial infection the keratin is broken down and degraded by the fungus) (*Rigopoulos* D *et al. 2017*)
- Beta-tubulin inhibition (main component of microtubules, essential for fungal cell mitosis)

<u>S. *aureus infection:*</u>

- Cytokeratin 15 (in case of fungal or bacterial infection the keratin is broken down and degraded by the bacterium) (*Aly R, 1996*)

The gene expression of the various markers was evaluated by quantitative relative RT-PCR (quantitative reverse transcription-polymerase chain reaction-qRT-PCR). This analysis involved 3 sequential steps:

- total RNA extraction;
- cDNA Reverse Transcription;
- qRT-PCR.

Total RNA was extracted from NCTC2544 cells according to what was described by Chomczynski and Mackey (1995).

At the end of the incubation with the active compounds of interest, the cells were washed with PBS (1 ×) and finally subjected to an RNA extraction procedure. At the end of the extraction, the extracted RNA was quantified using the QiaExpert instrument (Qiagen) and the concentrations of total RNA extracted at the wavelength of 260 nm were calculated in μg/mL.

Finally, the RNA integrity (2 μg/mL) was evaluated by means of an electrophoretic run on 1% agarose gel.

Total RNA was converted into cDNA (complementary DNA), using an enzyme capable of synthesizing a DNA molecule using a RNA strand as a template; this DNA polymerase-dependent RNA enzyme is called reverse transcriptase.

It binds to the 3' end of a single RNA strand and synthesizes the cDNA strand by means of random primers and deoxynucleotide triphosphate (DNTP).

For this purpose, a commercial kit "PrimeScript™ RT Reagent Kit (perfect Real Time)" (TakaraBioInc., Japan) containing 5X PrimeScript Buffer (for real Time); PrimeScript RT Enzyme Mix1; OligodTPrimer; Random 6 mers; RNAse free $dH_2O$ was used.

The extracted and quantified RNA was diluted to a concentration equal to 2 μg/mL and back-transcribed in cDNA.

A 10 μL Master Mix was prepared (containing 5X PrimeScript Buffer (for real Time); PrimeScript RT Enzyme Mix1; OligodTPrimer 50μM; Random 6 mers 100μM) to which 10 μL of RNA (2 μg/mL) were added. The samples were placed in a thermal cycler (Stratagene Mx3000P Real Time PCR System, Agilent Technologies Italia S.p.A., Milan, Italy) and subjected to reverse transcription under the following conditions:

37°C for 15 minutes; 85°C for 5 seconds;
4°C hold.

At the end of the reverse transcription, 30 μL of DEPC water were added to the samples to obtain a final cDNA concentration of 40 ng/μL.

qRT-PCR represents a method of amplification and quantification in real time of the amplified products by monitoring the fluorescence emitted during the reaction. The TaqMan® probe system (AppliedBiosystems) was used for RT-PCR amplification. The following TaqMan probes were used: Hs00174128_m1 *(TNF-α),* Hs00951967_g1 *(KRT15),* Hs00742533_s1e *(TUBB2A),* Hs01023894_m1 *(CDH1)* and Hs99999905_m1 (GAPDH). GAPDH was used as the housekeeping gene. The Taqman probe is a type of probe that allows the development of fluorescence as the amplification progresses. A reporter (FAM™ fluorophore) is linked to its 5' end, while a quencher is linked to its 3' end. The proximity between the reporter and the quencher cancels the emission of the fluorescence signal. The fluorescence is detected only with the 5' exonuclease activity of the thermostable DNA polymerase (Taq polymerase) and the accumulation of the amplification products can be evaluated by the increase in the fluorescence of the reporter which increases with each cycle.

For the qRT-PCR, a Master Mix was prepared as follows:

- 10 μL of "2X Premix Ex Taq" ;
- 1 μL of "20x TaqMan Gene ExpressionAssays" (containing 2 primers and the fluorescent probe labeled with FAM™ fluorophore);

- 0.4 μL of Rox II passive reference;
- 5 μL of DEPC water.

4 μL of cDNA for the target gene and 1 μL of cDNA for the housekeeping gene were added to the Master Mix. The amplification was carried out under the following conditions for 40 cycles:

- 95°C, 30 sec (Amplitaq activation);
- 95°C, 5 sec (Denaturation)
- 60°C, 20 sec (Annealing - extension); Each analysis was carried out in duplicate.

The data obtained were analyzed according to the $2^{-\Delta\Delta Ct}$ method and it was thus possible to calculate the relative expression values of the gene of interest, normalized with respect to the housekeeping gene and calibrated on the control sample (untreated cells):

$$\Delta\Delta Ct = \Delta Ct \text{ target-housekeeping(control)} - \Delta Ct \text{ target-housekeeping(treated cells)}$$

The $2^{-\Delta\Delta Ct}$ was calculated assuming an amplification efficiency of 100% (Vigetti *et* al., 2008)

**3.4.2 *IN VITRO* CELLULAR PROLIFERATION TEST ON HACAT CELLS**

The MTT assay (3-(4,5-dimethylthiazol-2-yl)-2,5-diphenyltetrazolium bromide) is a colorimetric assay used to evaluate *in vitro* cell proliferation, as it allows to measure proliferation and cell viability through the evaluation of mitochondrial activity (Mosmann *et al.,* 1983). This method is very useful to measure cell growth following treatment with mitogenic agents, antigenic stimuli, growth factors and for cytotoxicity studies.

The assay involves the use of a chromogenic oxidizing agent, MTT, consisting of a polycyclic system (C18H16BrN5S) bearing a tetrazole ring that can be easily reduced by mitochondrial dehydrogenases or other electronic transport systems, forming a nitrogenous chromogenic compound called formazan through opening of the tetrazole ring. Formazan forms crystals, insoluble in the intracellular environment, to which the membranes are substantially impermeable: the molecule can therefore enter the cell, but the product cannot exit if it has been correctly metabolized, that is if the electron transport chains are still metabolically active.

The formazan crystals are subsequently solubilized in dimethyl sulfoxide (DMSO), thus causing the solution to color change from yellow to dark blue-violet.

The assay was carried out following the method by Mosmann (1983), with some minor modifications. NCTC2544 human keratinocytes were seeded in a 96-well plate at a density of $5*10^4$ cells/well and incubated at 37°C, with 5% of $CO_2$, until reaching about 80% confluence.

Subsequently, the cells were incubated for 24 hours with the active compounds to be tested at the following concentrations: 1:1000/1:10000/1:100000/1:1000000 (according to the concentrations reported in Table 1). The products were tested in singles, pairs, and triplets.

The dilutions were prepared from stock in complete medium, sterile filtered, and using RPMI medium supplemented with 10% fetal bovine serum (FBS), in the presence of 1% penicillin and streptomycin.

Untreated cells were used as a positive control.

At the end of all treatments, the medium was collected and replaced with 100 μL of a 0.5 mg/mL MTT solution (Sigma-Aldrich, St. Louis, MO, USA) in complete culture medium.

After 3 hours of incubation at 37°C, the medium was collected and the formazan crystals were solubilized with 100 μL per well of DMSO (Sigma-Aldrich, St. Louis, MO, USA). The plate, covered with aluminum, was placed on a mechanical stirrer (Arhos 160 - PBI International, Milan, Italy) at 120 rpm for 15 minutes at room temperature.

The absorbance of the colored solution was measured using a spectrophotometric reader for microplates (BioTek Instruments Inc., BadFriedrichshall, Germany) at a wavelength of 570 nm (reference wavelength at 630 nm).

The data were expressed as a percentage of cell viability compared to control cells (ctr), according to the following formula:

$$\% \text{ cell viability / ctr} = (\text{Abs sample / Abs ctr}) * 100.$$

### 3.4.3 Anti-biofilm activity on S. *aureus*

The anti-biofilm activity of the tested compounds was evaluated as described by Miao *et al.,* 2019, as a reduction in the adhesion of the S. *aureus* biofilm. Fresh S. *aureus* was seeded at a density of $1*10^8$ CFU/well in 96-well flat bottom microplates, with or without the extracts and combinations thereof.

The plates were incubated, undisturbed, at 37°C per 24h. After 24h, the anti-biofilm activity was evaluated using the MTT cell viability test as reported in paragraph 3.3.5.

The data were expressed as a percentage of cell viability compared to control cells (Ctr), according to the following formula:

$$\% \text{ cell viability / ctr} = (\text{Abs sample / Abs ctr}) *100.$$

### 3.4.4 Anti-biofilm activity on C. *albicans*

The anti-biofilm activity of the tested compounds was evaluated as described by Kanchanapiboon *et al.,* 2020. The anti-biofilm activity was evaluated for all biofilm stages: adhesion, biofilm development, and mature biofilm (Figure 1).

To study the anti-biofilm adhesion activity, a fresh culture of C. *albicans* was seeded at a density of $5*10^6$ CFU/well in 96-well flat bottom microplates, with or without the extracts and combinations thereof. The plates were incubated, undisturbed, at 37°C for 90 minutes. After 90 minutes, the anti-biofilm activity was assessed using the MTT cell viability test as reported in paragraph 3.3.5. To study the biofilm growth inhibition activity, a fresh culture of C. *albicans* was seeded at a density of $5*10^6$ CFU/well in 96-well flat bottom microplates, with or without the extracts and combinations thereof. The plates were incubated, undisturbed, at 37°C for 90 minutes to promote cell adhesion and subsequently the non-adherent cells were removed by washing with PBS. At the end of the washings, incubation was carried out with the extracts and combinations thereof at 37°C for 24h. At the end of the incubation, the anti-biofilm activity was evaluated by means of the MTT cell viability test as reported in paragraph 3.3.5.

To study the inhibition activity on mature biofilm, a fresh culture of C. *albicans* was seeded at a density of $5*10^6$ CFU/well in 96-well flat bottom microplates and the cells were incubated, undisturbed, at 37°C for 24h to favor the biofilm development. At the end of 24h, the incubation with the extracts and combinations thereof was carried out for a further 24h. At the end of the incubation, the anti-biofilm activity was evaluated by means of the MTT cell viability test as reported in paragraph 3.3.5.

The viability data were expressed in all cases as a percentage of cell viability with respect to the control cells (Ctr), according to the following formula:

$$\% \text{ cell viability / ctr} = (\text{Abs sample / Abs ctr}) *100.$$

### 3.4.5 STATISTICAL ANALYSIS

The statistical analysis was carried out by Student's t test using the Graphpad software (version 7.00 for Windows, GraphPad Software, La Jolla California USA, www.graphpad.com)

## 4. RESULTS

4.1 In vitro in a HACAT cell co-infection model with *C. albicans* (Kanchanapibon et *al.,* 2020) or S. *aureus*

Figure 1 shows the gene expression data of the TNF-alpha marker after 24 hours of incubation with the compounds under analysis and *C. albicans.*

In the positive control (cells treated with *C. albicans*) there is a significant increase in TNF-alpha.

The greatest reduction in *TNF-alpha* gene expression is shown in the presence of treatment with TROSYD ACTIVE MIX3 COMBINATION (1:1000) and this effect is found to be synergistic with respect to the other combinations tested.

Fig. 2 illustrates bar graphs relating to TNF-$\alpha$ gene expression in HACAT cells co-infected with *C. albicans* and treated with the composition containing the three active components, Spilanthes, cocoa and zinc, and with the individual components.

TNF-$\alpha$ gene expression in HACAT cells was evaluated by qRT-PCR. The cells were co-infected with *C. albicans* and treated with the extracts in singles, pairs, or triplets, and incubated at 37°C for 24h, 5% $CO_2$. Complete RPMI (Control); RPMI with *C. albicans* (Positive Control). The values represent the Mean$\pm$SEM of two experiments carried

out in duplicate.

*($p<0.05$).

Figure 3 shows the gene expression data of the E-cadherin marker after 24 hours of incubation with the compounds under analysis and *C. albicans.*

In the positive control (cells treated with C. *albicans*) there was a significant decrease in E-cadherin. The greatest increase in *E-cadherin* gene expression is evident in the presence of treatment with TROSYD ACTIVE MIX3 COMBINATION (1:1000) and this effect is found to be synergistic with respect to the other combinations tested.

In particular, Fig. 3 illustrates a graph relating to *E-cadherin* gene expression. E-*cadherin* gene expression in HACAT cells was evaluated by qRT-PCR. The cells were co-infected with *C. albicans* and treated with the extracts in singles, pairs or triplets and incubated at 37°C for 24h, 5% $CO_2$. Complete RPMI (Control); RPMI with *C. albicans* (Positive Control). The values represent the Mean±SEM of two experiments carried out in duplicate.

*($p<0.05$), **($p<0.01$).

Figure 4 shows gene expression data of the KRT15 marker (Keratin 15) after 24 hours of incubation with the compounds under analysis and *C. albicans.*

In the positive control (cells treated with *C. albicans*) there is a significant decrease in KRT15. The greatest and significant increase in the *KRT15* gene expression is evident in the presence of treatment with MIX3 combination (1:1000) and this effect is found to be synergistic with respect to the other combinations tested.

In particular, Fig. 4 illustrates a graph relating to *KRT15* gene expression. *KRT15* gene expression in HACAT cells was evaluated by qRT-PCR. The cells were co-infected with *C. albicans* and treated with the extracts in singles, pairs or triplets and incubated at 37°C for 24h, 5% $CO_2$. Complete RPMI (Control); RPMI with C. *albicans* (Positive Control). The values represent the Mean±SEM of two experiments carried out in duplicate.

*($p<0.05$), **($p<0.01$).

Figure 5 shows the gene expression data of the Beta-tubulin marker after 24 hours of incubation with the compounds under analysis and *C. albicans.*

In the positive control (cells treated with *C. albicans)* there is a significant decrease in Beta-tubulin.

The greatest and significant reduction in *Beta-tubulin* gene expression is evident in the presence of treatment with the three-component combination of the invention (1:1000) and this effect is found to be synergistic with respect to the other combinations tested.

In particular, Fig. 5 illustrates a graph relating to *Beta-tubulin* gene expression. *Beta-tubulin* gene expression in HACAT cells was evaluated by qRT-PCR. The cells were co-infected with *C. albicans* and treated with the extracts in singles, pairs or triplets and incubated at 37°C for 24h, 5% $CO_2$. Complete RPMI (Control); RPMI with *C. albicans* (Positive Control). The values represent the Mean±SEM of two experiments carried out in duplicate. *($p<0.05$), **($p<0.01$).

Figure 6 shows C. *albicans* growth in the presence of the extracts and the combination thereof. The curves are obtained from the measurement of the area ($\mu m^2$) covered by *C. albicans* at T0, after 120 min, and after 1440 minutes.

The greatest reduction in the area covered by *C. albicans* is evident in the presence of the treatment with the combination of the invention (1:1000) and this effect is found to be synergistic with respect to the other combinations tested.

In particular, in the attached Fig. 6, the C. *albicans* growth is expressed as the area ($\mu m^2$ covered by the fungus. The images were acquired at T0, after 120 min, and after 1440 min, by means of an inverted phase microscope at 4X magnification.

Figure 7 shows the *KRT15* marker gene expression data after 24 hours of incubation with the compounds under analysis and S. *aureus.*

In the positive control (cells treated with S. *aureus*) there is a significant increase in KRT15.

The greatest reduction in *KRT15* gene expression is evident in the presence of treatment with the three-component combination (1:1000) and this effect is found to be synergistic with respect to the other combinations tested.

In particular, Fig. 7 illustrates a representative graph of *KRT15* gene expression. *KRT15* gene expression in HACAT cells was evaluated by qRT-PCR. The cells were co-infected with S. *aureus* and treated with the extracts in singles, pairs or triplets and incubated at 37°C for 24h, 5% $CO_2$. Complete RPMI (Control); RPMI with S. *aureus* (Positive Control). The values represent the Mean±SEM of two experiments carried out in duplicate. *($p<0.05$), **($p<0.01$).

<u>*4.2 In vitro* cell proliferation test on HACAT cells</u>

Figures 8 and 9 show the proliferation data at 24 and 48 hours, respectively, in Hacat cells treated with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL). The extracts were treated in pairs and triplets in a 15:4:1 ratio at all tested concentrations. Untreated cells represent the negative control.

After 24h of treatment (Figure 8) none of the extracts at any of the tested concentrations and combinations have a cytotoxic effect. At 48h (Fig. 9), the highest concentrations of Cocoa (16μg/mL) and Zinc (4μg/mL) exhibit a

significant ($p<0.05$) cytotoxic activity on HACAT cells. This is also true for the two highest combinations of Spilanthes and Zinc (Figure 9).

After 24h of treatment there was a significant ($p<0.05$) synergistic effect of the extracts at all dilutions tested compared to single extracts and combinations in pairs (Figure 8).

After 48h of treatment there is a significant ($p<0.05$) synergistic effect of the extracts at the most concentrated dilutions (1:1000 and 1:10000) as compared to the single extracts and the combinations in pairs (Figure 9).

In particular, Fig.8 illustrates bar graphs representative of cell viability at 24h. % of cell viability on HACAT cells following treatment for 24 hours with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL). The extracts were treated in pairs and triplets in a 15:4:1 ratio at all tested concentrations. Untreated Cells (Control). *($p<0.05$), **($p<0.01$).

| COMBINATIONS | Student’s t-test |
|---|---|
| Trosyd active MIX3 (1:1000000) | |
| Spilanthes 60ng/mL | * |
| Cocoa 16ng/mL | * |
| Zinc 4ng/mL | ** |
| Spilanthes 60ng/mL+ Cocoa 16ng/mL | * |
| Spilanthes 60ng/mL+ Zinc 4ng/mL | * |
| Cocoa 16ng/mL + Zinc 4ng/mL | * |

| COMBINATIONS | Student’s t-test |
|---|---|
| Trosyd active MIX3 (1:100000) | |
| Spilanthes 600ng/mL | *** |
| Cocoa 160ng/mL | ** |
| Zinc 40ng/mL | ** |
| Spilanthes 600ng/mL+ Cocoa 160ng/mL | ** |
| Spilanthes 600ng/mL+ Zinc 40ng/mL | ** |
| Cocoa 160ng/mL + Zinc 40ng/mL | ** |

| COMBINATIONS | Student’s t-test |
|---|---|
| Trosyd active MIX3 (1:10000) | |
| Spilanthes 6μg/mL | * |
| Cocoa 1.6μg/mL | * |
| Zinc 400ng/mL | * |
| Spilanthes 6μg/mL+ Cocoa 1.6μg/mL | * |
| Spilanthes 6μg/mL+ Zinc 400ng/mL | ** |
| Cocoa 1.6μg/mL+ Zinc 400ng/mL | ** |

| COMBINATIONS | Student’s t-test |
|---|---|
| Trosyd active MIX3 (1:1000) | |
| Spilanthes 60μg/mL | * |

(continued)

| COMBINATIONS | Student's t-test |
|---|---|
| Cocoa 16μg/mL | ** |
| Zinc 4μg/mL | * |
| Spilanthes 60μg/mL+ Cocoa 16μg/mL | ** |
| Spilanthes 60μg/mL+ Zinc 4μg/mL | * |
| Cocoa 16μg/mL+ Zinc 4μg/mL | ** |

In particular, Fig. 9 illustrates bar graphs representative of cellular viability at 48h. % of cell viability in HACAT cells following treatment for 24 hours with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL).The extracts were treated in pairs and triplets in a 15:4:1 ratio at all tested concentrations. Untreated Cells (Control). *($p<0.05$), **($p<0.01$), ***($p<0.005$)

| COMBINATIONS | Student's t-test |
|---|---|
| Trosyd active MIX3 (1:1000000) | |
| Spilanthes 60ng/mL | * |
| Cocoa 16ng/mL | * |
| Zinc 4ng/mL | * |
| Spilanthes 60ng/mL+ Cocoa 16ng/mL | * |
| Spilanthes 60ng/mL+ Zinc 4ng/mL | |
| Cocoa 16ng/mL + Zinc 4ng/mL | |

| COMBINATIONS | Student's t-test |
|---|---|
| Trosyd active MIX3 (1:100000) | |
| Spilanthes 600ng/mL | ** |
| Cocoa 160ng/mL | ** |
| Zinc 40ng/mL | ** |
| Spilanthes 600ng/mL+ Cocoa 160ng/mL | ** |
| Spilanthes 600ng/mL+ Zinc 40ng/mL | ** |
| Cocoa 160ng/mL + Zinc 40ng/mL | ** |

| COMBINATIONS | Student's t-test |
|---|---|
| Trosyd active MIX3 (1:10000) | |
| Spilanthes 6μg/mL | ** |
| Cocoa 1.6μg/mL | ** |
| Zinc 400ng/mL | ** |
| Spilanthes 6μg/mL+ Cocoa 1.6μg/mL | ** |
| Spilanthes 6μg/mL+ Zinc 400ng/mL | ** |
| Cocoa 1.6μg/mL+ Zinc 400ng/mL | ** |

| COMBINATIONS | Student's t-test |
|---|---|
| Trosyd active MIX3 (1:1000) | |
| Spilanthes 60μg/mL | * |
| Cocoa 16μg/mL | *** |
| Zinc 4μg/mL | *** |
| Spilanthes 60μg/mL+ Cocoa 16μg/mL | * |
| Spilanthes 60μg/mL+ Zinc 4μg/mL | *** |
| Cocoa 16μg/mL+ Zinc 4μg/mL | * |

4.3Anti-biofilm activity on S. *aureus*

Figure 10 shows the adhesion data of S. *aureus* biofilm (expressed as % viability as compared to the untreated control) under co-treatment with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL). The extracts were treated in pairs and triplets in a 15:4:1 ratio at all tested concentrations. Untreated cells represent the negative control. A decrease in cell viability corresponds to a greater activity in contrasting the adhesion of S. *aureus* biofilm.

There is a synergistic effect with the combination of the three components (1:1000) although all the other treatments exhibit significant data as compared to the control ($p<0.05$).

In particular, Fig. 10 illustrates bar graphs representative of S. *aureus* biofilm adhesion under co-treatment for 24 hours with Spilanthes (60ng/mL; 6ng/mL; 6μg/mL; 60μg/mL); Cocoa (16ng/mL; 160ng/mL; 16μg/mL; 16μg/mL) and Zinc (4ng/mL; 40ng/mL; 400ng/mL; 4μg/mL). The extracts were treated in pairs and triplets in a 15:4:1 ratio at all tested concentrations. Untreated cells (Control). *($p<0.05$), **($p<0.01$), ***($p<0.005$).

| COMBINATIONS | Student's t-test |
|---|---|
| Trosyd active MIX3 (1:1000000) | |
| Spilanthes 60ng/mL | |
| Cocoa 16ng/mL | |
| Zinc 4ng/mL | * |
| Spilanthes 60ng/mL+ Cocoa 16ng/mL | |
| Spilanthes 60ng/mL+ Zinc 4ng/mL | |
| Cocoa 16ng/mL + Zinc 4ng/mL | |

| COMBINATIONS | Student's t-test |
|---|---|
| Trosyd active MIX3 (1:100000) | |
| Spilanthes 600ng/mL | * |
| Cocoa 160ng/mL | * |
| Zinc 40ng/mL | |
| Spilanthes 600ng/mL+ Cocoa 160ng/mL | |
| Spilanthes 600ng/mL+ Zinc 40ng/mL | * |
| Cocoa 160ng/mL + Zinc 40ng/mL | |

| COMBINATIONS | Student's t-test |
|---|---|
| Trosyd active MIX3 (1:10000) | |
| Spilanthes 6μg/mL | |
| Cocoa 1.6μg/mL | |
| Zinc 400ng/mL | |
| Spilanthes 6μg/mL+ Cocoa 1.6μg/mL | |
| Spilanthes 6μg/mL+ Zinc 400ng/mL | * |
| Cocoa 1.6μg/mL+ Zinc 400ng/mL | |

| COMBINATIONS | Student's t-test |
|---|---|
| Trosyd active MIX3 (1:1000) | |
| Spilanthes 60μg/mL | * |
| Cocoa 16μg/mL | * |
| Zinc 4μg/mL | * |
| Spilanthes 60μg/mL+ Cocoa 16μg/mL | * |
| Spilanthes 60μg/mL+ Zinc 4μg/mL | * |
| Cocoa 16μg/mL+ Zinc 4μg/mL | * |

4.4Anti-biofilm activity (adhesion, development and mature biofilm) on C. *albicans*

Figure 11 shows the adhesion data of C. *albicans* biofilm (expressed as % viability as compared to the untreated control) under co-treatment with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL).The extracts were treated in pairs and triplets in a 15:4:1 ratio at all tested concentrations. Untreated cells represent the negative control. A decrease in cell viability corresponds to a greater activity in contrasting the adhesion of C. *albicans* biofilm.

A synergistic effect is found only with the combination or MIX3 (1:1000) although all other treatments exhibit significant data as compared to the control (p<0.05).

In particular, Fig. 11 illustrates bar graphs representative of C. *albicans* biofilm adhesion. % adhesion of C. *albicans* biofilm under co-treatment for 90 minutes with Spilanthes (60ng/mL; 6ng/mL; 6μg/mL; 60μg/mL); Cocoa (16ng/mL; 160ng/mL; 1.6μg/mL,16μg/mL) and Zinc (4ng/mL; 40ng/mL; 400ng/mL; 4μg/mL). The extracts were treated in pairs and triplets in a 15:4:1 ratio at all tested concentrations. Untreated Cells (Control). *(p<0.05), **(p<0.01), ***(p<0.005).

| COMBINATIONS | Student's t-test |
|---|---|
| Trosyd active MIX3 (1:1000000) | |
| Spilanthes 60ng/mL | * |
| Cocoa 16ng/mL | |
| Zinc 4ng/mL | |
| Spilanthes 60ng/mL+ Cocoa 16ng/mL | |
| Spilanthes 60ng/mL+ Zinc 4ng/mL | |
| Cocoa 16ng/mL + Zinc 4ng/mL | |

| COMBINATIONS | Student's t-test |
|---|---|
| Trosyd active MIX3 (1:100000) | |
| Spilanthes 600ng/mL | * |
| Cocoa 160ng/mL | |
| Zinc 40ng/mL | |
| Spilanthes 600ng/mL+ Cocoa 160ng/mL | |
| Spilanthes 600ng/mL+ Zinc 40ng/mL | |
| Cocoa 160ng/mL + Zinc 40ng/mL | |

| COMBINATIONS | Student's t-test |
|---|---|
| Trosyd active MIX3 (1:10000) | |
| Spilanthes 6μg/mL | * |
| Cocoa 1.6μg/mL | |
| Zinc 400ng/mL | |
| Spilanthes 6μg/mL+ Cocoa 1.6μg/mL | |
| Spilanthes 6μg/mL+ Zinc 400ng/mL | |
| Cocoa 1.6μg/mL+ Zinc 400ng/mL | |

| COMBINATIONS | Student's t-test |
|---|---|
| Trosyd active MIX3 (1:1000) | |
| Spilanthes 60μg/mL | ** |
| Cocoa 16μg/mL | ** |
| Zinc 4μg/mL | |
| Spilanthes 60μg/mL+ Cocoa 16μg/mL | * |
| Spilanthes 60μg/mL+ Zinc 4μg/mL | * |
| Cocoa 16μg/mL+ Zinc 4μg/mL | * |

Figure 12 shows the development data of C. *albicans* biofilm (expressed as % viability as compared to the untreated control) under co-treatment with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL).The extracts were treated in pairs and triplets in a 15:4:1 ratio at all tested concentrations. Untreated cells represent the negative control. A decrease in cell viability corresponds to a greater activity in contrasting C. *albicans* biofilm development.

A synergistic effect is found only with the COMBINATION of the three-component mixture, (1:1000) although all the other treatments exhibit significant data as compared to the control ($p<0.05$).

In particular, Fig. 12 illustrates bar graphs representative of C. *albicans* biofilm development. % of C. *albicans* biofilm development under co-treatment for 90 minutes with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL). The extracts were treated in pairs and triplets in a 15:4:1 ratio at all tested concentrations. Untreated cells (Control). *($p<0.05$), **($p<0.01$), ***($p<0.005$).

| COMBINATIONS | Student's t-test |
|---|---|
| Trosyd active MIX3 (1:1000000) | |
| Spilanthes 60ng/mL | |

(continued)

| COMBINATIONS | Student's t-test |
|---|---|
| Cocoa 16ng/mL | * |
| Zinc 4ng/mL | * |
| Spilanthes 60ng/mL+ Cocoa 16ng/mL | * |
| Spilanthes 60ng/mL+ Zinc 4ng/mL | * |
| Cocoa 16ng/mL + Zinc 4ng/mL | * |

| COMBINATIONS | Student's t-test |
|---|---|
| Trosyd active MIX3 (1:100000) | |
| Spilanthes 600ng/mL | * |
| Cocoa 160ng/mL | |
| Zinc 40ng/mL | |
| Spilanthes 600ng/mL+ Cocoa 160ng/mL | |
| Spilanthes 600ng/mL+ Zinc 40ng/mL | |
| Cocoa 160ng/mL + Zinc 40ng/mL | |

| COMBINATIONS | Student's t-test |
|---|---|
| Trosyd active MIX3 (1:10000) | |
| Spilanthes 6μg/mL | * |
| Cocoa 1.6μg/mL | * |
| Zinc 400ng/mL | * |
| Spilanthes 6μg/mL+ Cocoa 1.6μg/mL | * |
| Spilanthes 6μg/mL+ Zinc 400ng/mL | * |
| Cocoa 1.6μg/mL+ Zinc 400ng/mL | |

| COMBINATIONS | Student's t-test |
|---|---|
| Trosyd active MIX3 (1:1000) | |
| Spilanthes 60μg/mL | * |
| Cocoa 16μg/mL | * |
| Zinc 4μg/mL | * |
| Spilanthes 60μg/mL+ Cocoa 16μg/mL | ** |
| Spilanthes 60μg/mL+ Zinc 4μg/mL | ** |
| Cocoa 16μg/mL+ Zinc 4μg/mL | ** |

Figure 13 shows the development data of C. *albicans* biofilm (expressed as % viability as compared to the untreated control) under co-treatment with Spilanthes (60ng/mL; 6ng/mL; 6μg/mL; 60μg/mL); Cocoa (16ng/mL; 160ng/mL; 1.6μg/mL; 16μg/mL) and Zinc (4ng/mL; 40ng/mL; 400ng/mL; 4μg/mL). The extracts were treated in pairs and triplets in a 15:4:1 ratio at all tested concentrations. Untreated cells represent the negative control. A decrease in

cell viability corresponds to a greater activity in contrasting the development of C. *albicans* biofilm.

A synergistic effect is found only with the combination of the invention (1:1000) although all other treatments exhibit significant data as compared to the control ($p<0.05$).

In particular, Fig.13 illustrates bar graphs representative of the development of mature *C. albicans* biofilm. % viability of mature *C. albicans* biofilm under co-treatment for 90 minutes with Spilanthes (60ng/mL; 6ng/mL; 6μg/mL; 60μg/mL); Cocoa (16ng/mL; 160ng/mL; 1.6μg/mL, 16μg/mL) and Zinc (4ng/mL; 40ng/mL; 400ng/mL; 4μg/mL). The extracts were treated in pairs and triplets in a 15:4:1 ratio at all tested concentrations. Untreated cells (Control). *($p<0.05$), **($p<0.01$), ***($p<0.005$).

| COMBINATIONS | Student's t-test |
|---|---|
| Trosyd active MIX3 (1:1000000) | |
| Spilanthes 60ng/mL | |
| Cocoa 16ng/mL | * |
| Zinc 4ng/mL | * |
| Spilanthes 60ng/mL+ Cocoa 16ng/mL | * |
| Spilanthes 60ng/mL+ Zinc 4ng/mL | * |
| Cocoa 16ng/mL + Zinc 4ng/mL | * |

| COMBINATIONS | Student's t-test |
|---|---|
| Trosyd active MIX3 (1:100000) | |
| Spilanthes 600ng/mL | |
| Cocoa 160ng/mL | |
| Zinc 40ng/mL | |
| Spilanthes 600ng/mL+ Cocoa 160ng/mL | |
| Spilanthes 600ng/mL+ Zinc 40ng/mL | |
| Cocoa 160ng/mL + Zinc 40ng/mL | |

| COMBINATIONS | Student's t-test |
|---|---|
| Trosyd active MIX3 (1:10000) | |
| Spilanthes 6μg/mL | * |
| Cocoa 1.6μg/mL | * |
| Zinc 400ng/mL | |
| Spilanthes 6μg/mL+ Cocoa 1.6μg/mL | * |
| Spilanthes 6μg/mL+ Zinc 400ng/mL | * |
| Cocoa 1.6μg/mL+ Zinc 400ng/mL | * |

| COMBINATIONS | Student's t-test |
|---|---|
| Trosyd active MIX3 (1:1000) | |
| Spilanthes 60μg/mL | * |
| Cocoa 16μg/mL | * |
| Zinc 4μg/mL | * |

(continued)

| COMBINATIONS | Student's t-test |
|---|---|
| **Spilanthes 60$\mu$g/mL+ Cocoa 16$\mu$g/mL** | * |
| **Spilanthes 60$\mu$g/mL+ Zinc 4$\mu$g/mL** | * |
| **Cocoa 16$\mu$g/mL+ Zinc 4$\mu$g/mL** | * |

## Claims

1. A composition comprising a combination of spilanthol, or a *Spilanthes acmella* extract which contains it, with zinc or a salt thereof, preferably zinc bisglycinate, and cocoa.

2. The composition according to claim 1, wherein the *Spilanthes acmella* extract is obtained by extraction of the plant inflorescence with a polar protic solvent preferably selected from water, methanol, ethanol, and mixtures thereof, or an apolar solvent preferably hexane.

3. The composition according to claim 1 or 2 comprising a further biologically active ingredient selected from selenium, valine optionally in the form of a salt or an ester, vitamin D preferably vitamin D3, ornithine optionally in the form of a salt or an ester, and mixtures thereof.

4. The composition according to any one of claims 1-3 comprising spilanthol, or a *Spilanthes acmella* extract which contains it, zinc or a salt or oxide thereof, cocoa, selenium, valine optionally in the form of a salt or an ester, vitamin D preferably vitamin D3, ornithine optionally in the form of a salt or an ester.

5. A food supplement comprising a composition according to any one of claims 1-4 and a physiologically acceptable carrier.

6. Non-therapeutic use of the composition according to any one of claims 1-4, or the food supplement according to claim 5, for improving the aesthetic appearance of the nails and/or for treating fractures, flaking, cracks, dulling of the nail and/or for accelerating nail regrowth.

7. The composition according to any one of claims 1-4, for oral or topical use in the prevention or treatment of a nail infection of bacterial origin, in particular from *Staphylococcus aureus,* or of fungal origin, in particular from *Candida albicans.*

8. The composition according to any one of claims 1-4, for oral or topical use in the prevention or treatment of nail trauma or dystrophy.

9. The composition according to any one of claims 1-4, for oral or topical use in the prevention or treatment of inflammation of the nail and of the surrounding skin tissues and appendages on which the nail develops.

10. The composition according to any one of claims 1-4, for oral or topical use in the prevention or treatment of psoriasis of the nail and of the surrounding skin tissues and appendages on which the nail develops.

FIG. 1

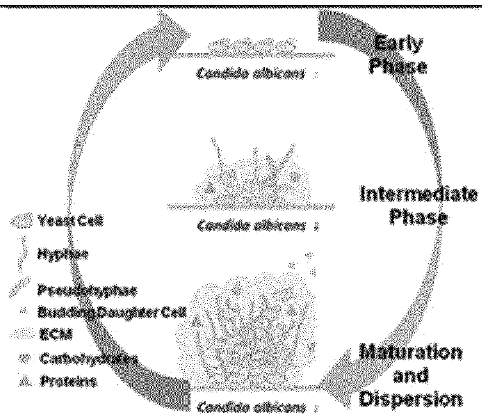
Early Phase
Intermediate Phase
Maturation and Dispersion
Candida albicans
Candida albicans
Candida albicans
Yeast Cell
Hyphae
Pseudohyphae
Budding Daughter Cell
ECM
Carbohydrates
Proteins

FIG. 2

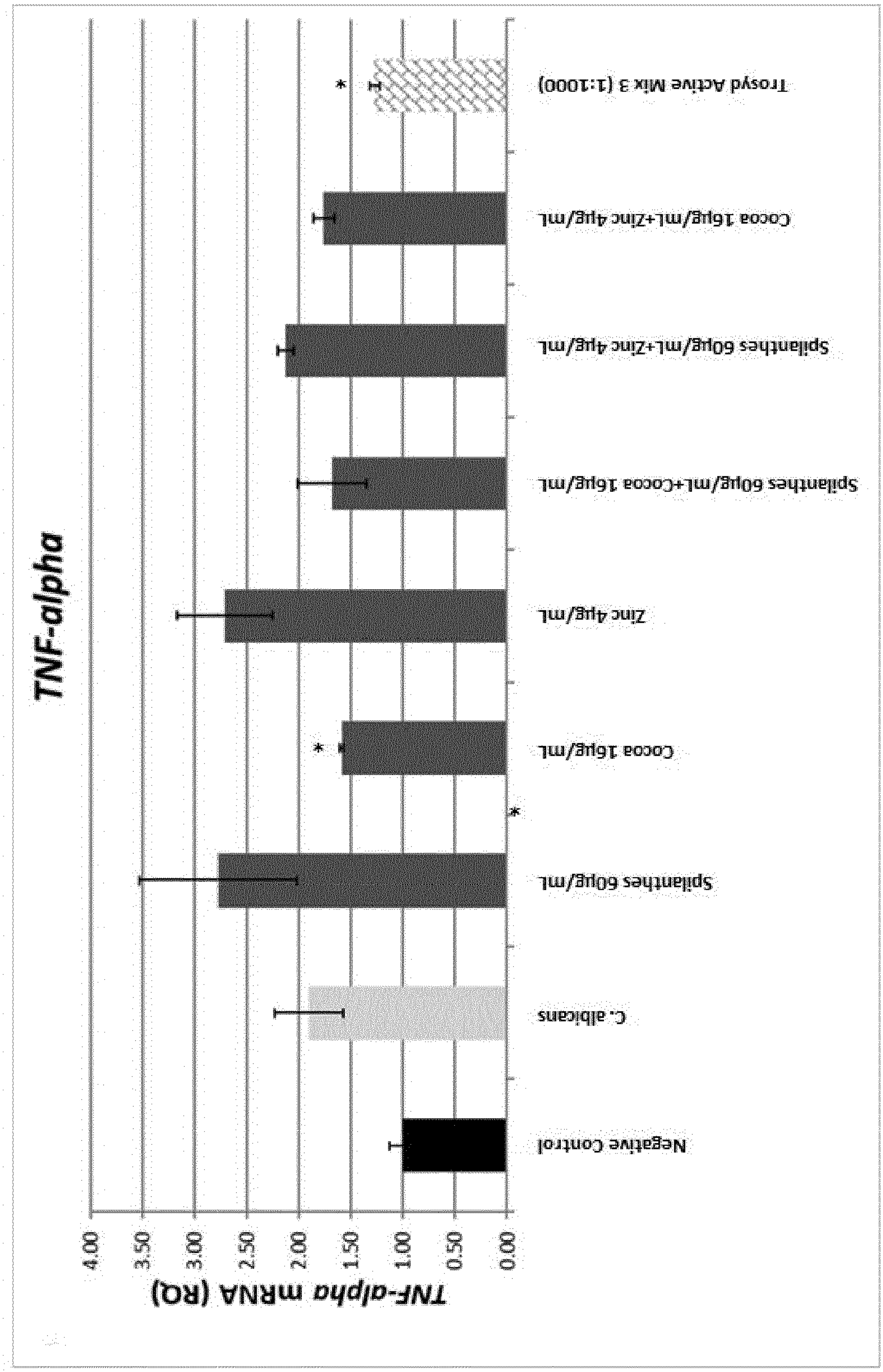
TNF-alpha
TNF-alpha mRNA (RQ)
0.00
0.50
1.00
1.50
2.00
2.50
3.00
3.50
4.00
Negative Control
C. albicans
Spilanthes 60µg/mL
Cocoa 16µg/mL
Zinc 4µg/mL
Spilanthes 60µg/mL+Cocoa 16µg/mL
Spilanthes 60µg/mL+Zinc 4µg/mL
Cocoa 16µg/mL+Zinc 4µg/mL
Trosyd Active Mix 3 (1:1000)
*

FIG. 3

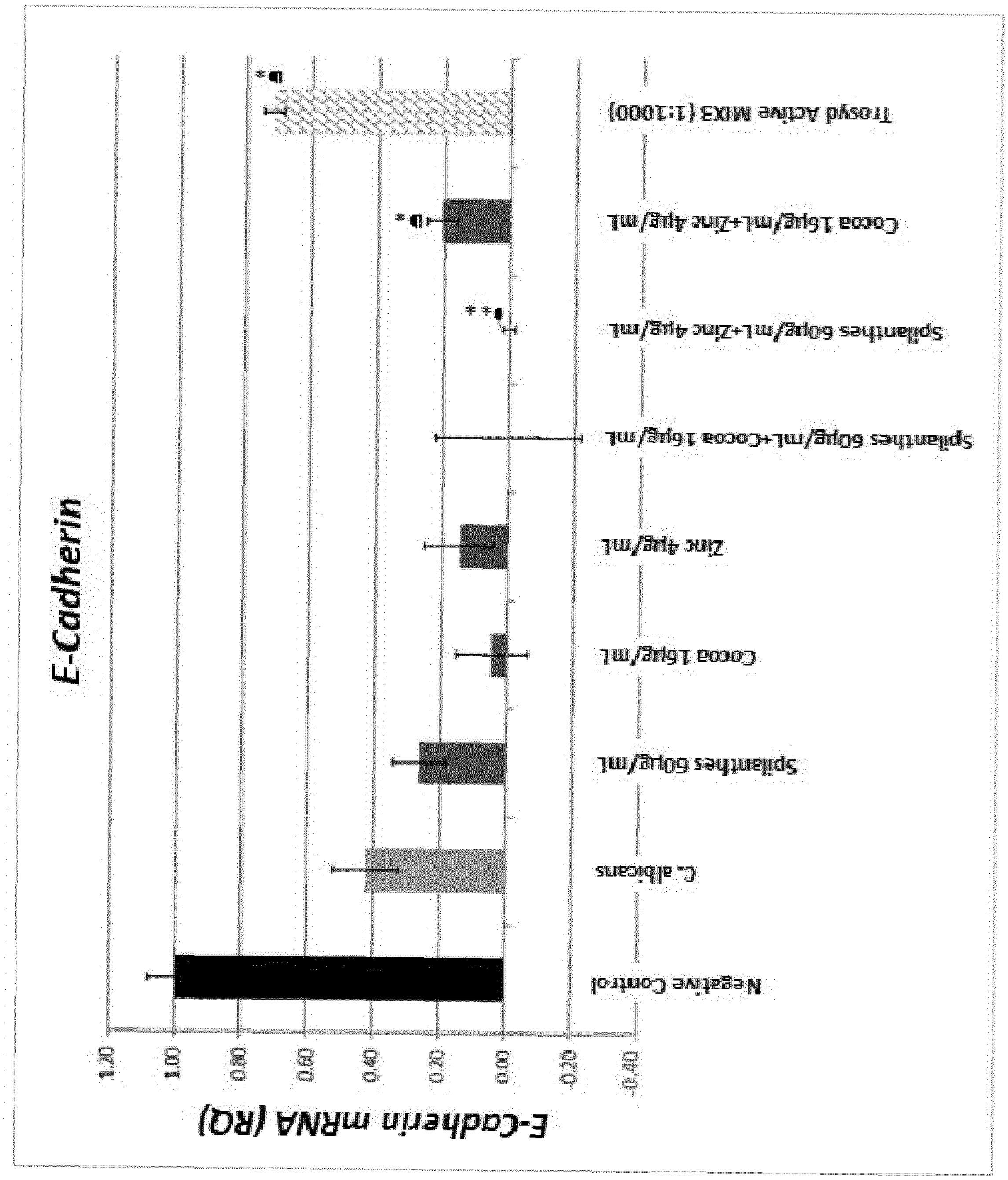
E-Cadherin
E-Cadherin mRNA (RQ)
1.20
1.00
0.80
0.60
0.40
0.20
0.00
-0.20
-0.40
Negative Control
C. albicans
Spilanthes 60µg/ml
Cocoa 16µg/ml
Zinc 4µg/mL
Spilanthes 60µg/mL+Cocoa 16µg/mL
Spilanthes 60µg/mL+Zinc 4µg/mL
Cocoa 16µg/mL+Zinc 4µg/mL
Trosyd Active MIX3 (1:1000)

FIG. 4

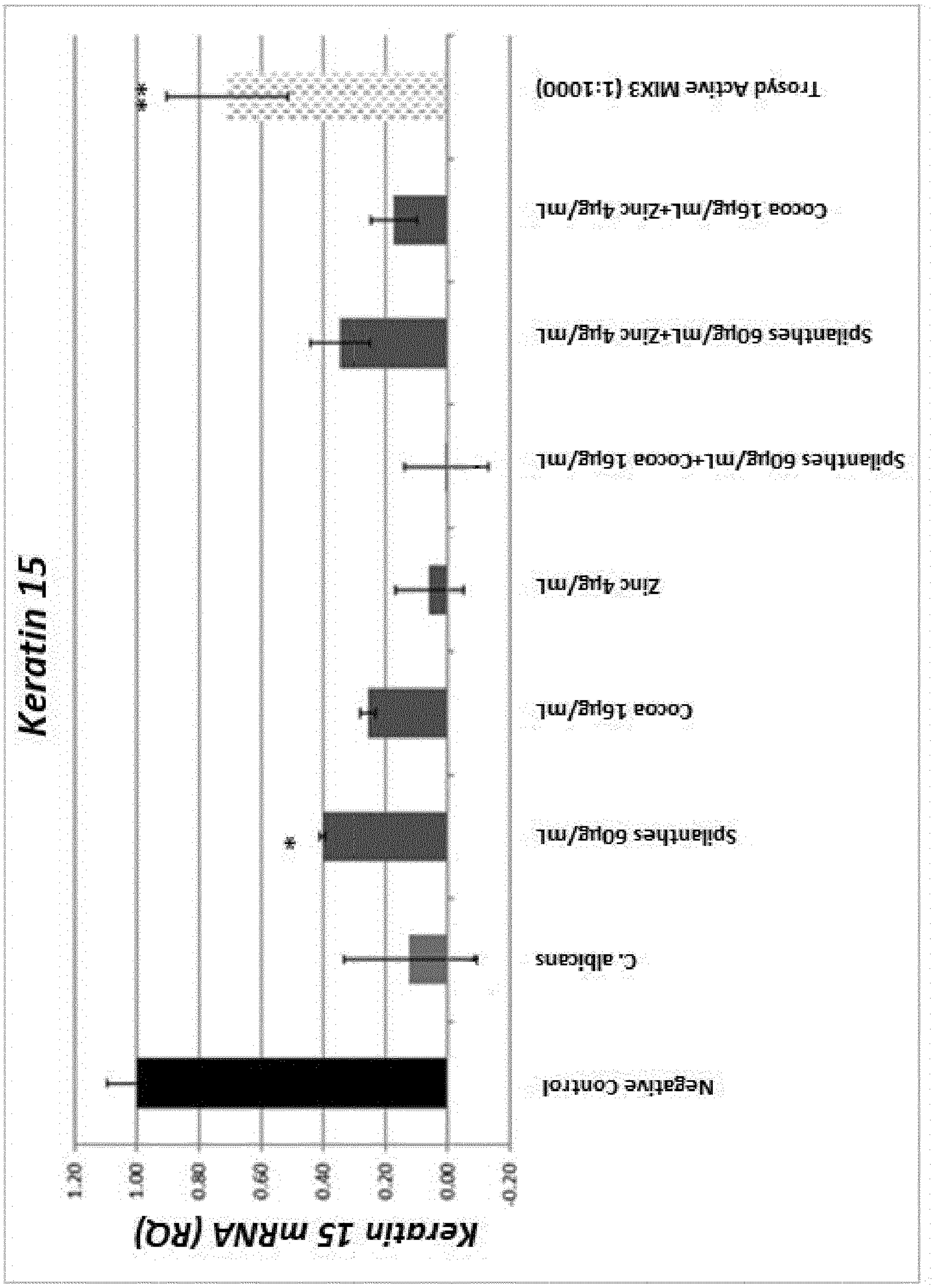
Keratin 15
Keratin 15 mRNA (RQ)
1.20
1.00
0.80
0.60
0.40
0.20
0.00
-0.20
Negative Control
C. albicans
Spilanthes 60ug/mL
Cocoa 16ug/mL
Zinc 4ug/mL
Spilanthes 60ug/mL+Cocoa 16ug/mL
Spilanthes 60ug/mL+Zinc 4ug/mL
Cocoa 16ug/mL+Zinc 4ug/mL
Trosyd Active MIX3 (1:1000)
*
**

FIG. 5

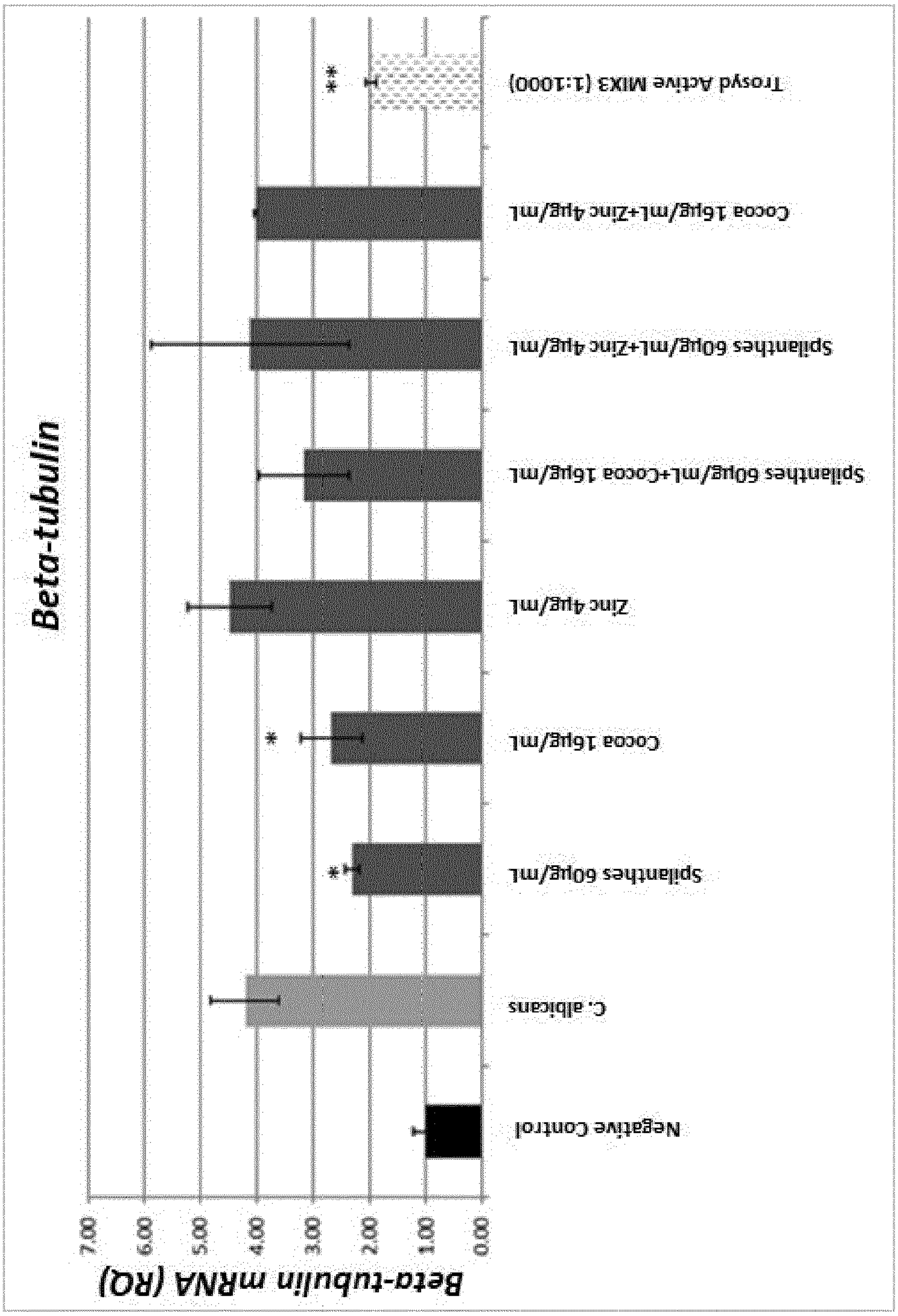
Beta-tubulin
Beta-tubulin mRNA (RQ)
7.00
6.00
5.00
4.00
3.00
2.00
1.00
0.00
Negative Control
C. albicans
Spilanthes 60µg/mL
Cocoa 16µg/mL
Zinc 4µg/mL
Spilanthes 60µg/mL+Cocoa 16µg/mL
Spilanthes 60µg/mL+Zinc 4µg/mL
Cocoa 16µg/mL+Zinc 4µg/mL
Trosyd Active MIX3 (1:1000)
*
*
**

FIG. 6

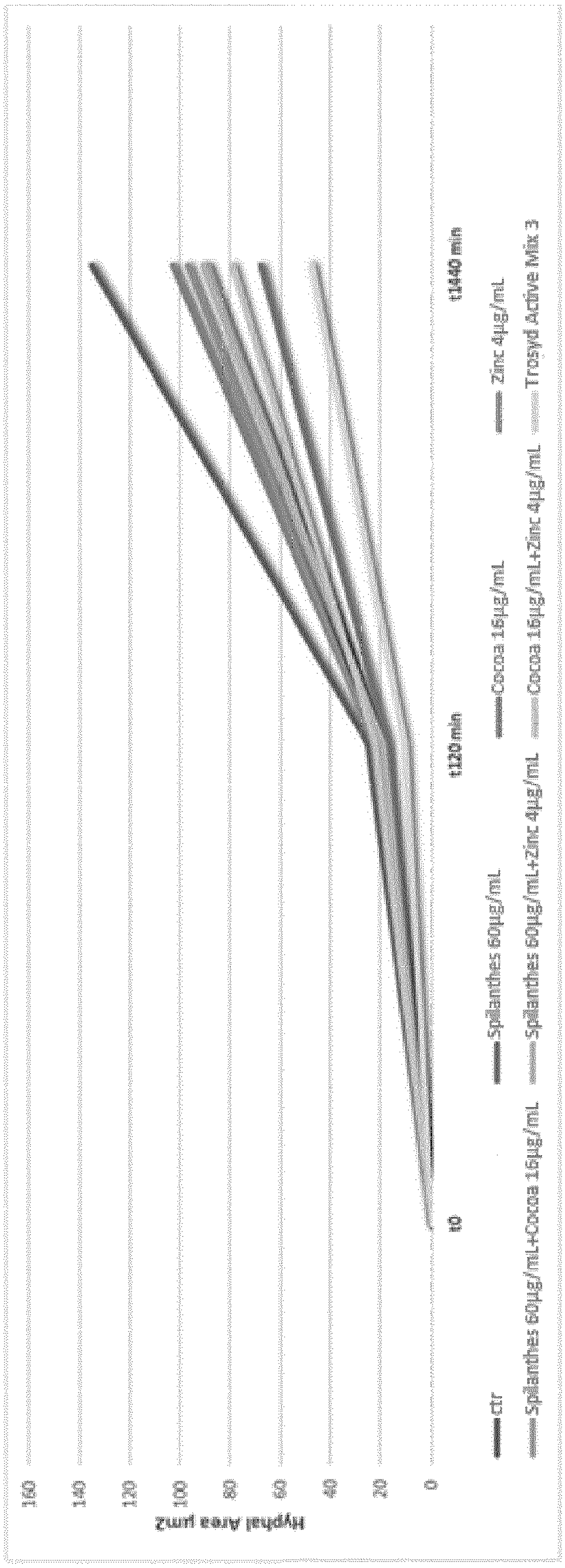
Hyphal Area µm2
160
140
120
100
80
60
40
20
0
t0
t120 min
t1440 min
ctr
Spilanthes 60µg/mL
Cocoa 16µg/mL
Zinc 4µg/mL
Spilanthes 60µg/mL+Cocoa 16µg/mL
Spilanthes 60µg/mL+Zinc 4µg/mL
Cocoa 16µg/mL+Zinc 4µg/mL
Trosyd Active Mix 3

FIG. 7

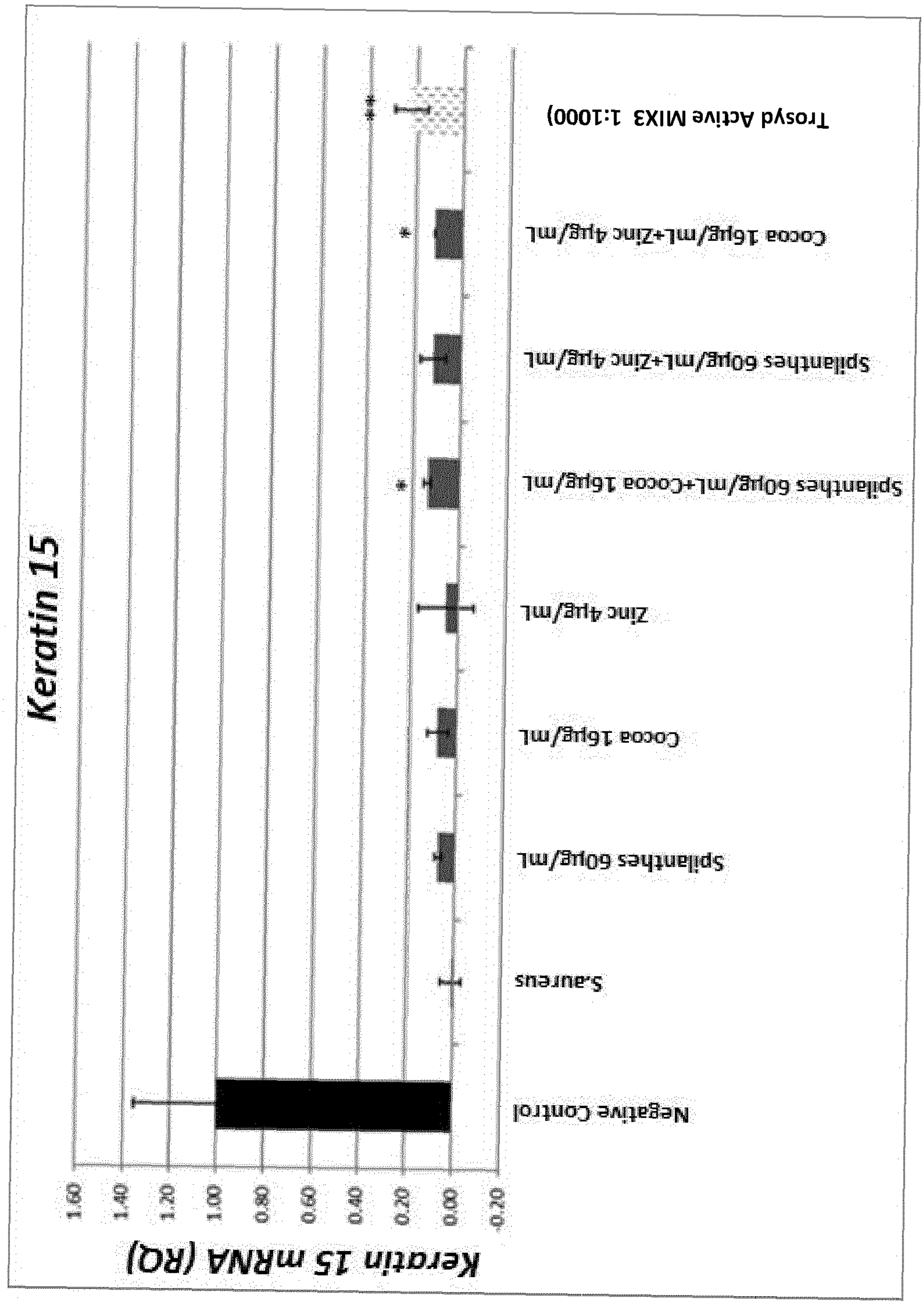
Keratin 15
Keratin 15 mRNA (RQ)
1.60
1.40
1.20
1.00
0.80
0.60
0.40
0.20
0.00
-0.20
Negative Control
S.aureus
Spilanthes 60µg/mL
Cocoa 16µg/mL
Zinc 4µg/mL
Spilanthes 60µg/mL+Cocoa 16µg/mL
Spilanthes 60µg/mL+Zinc 4µg/mL
Cocoa 16µg/mL+Zinc 4µg/mL
Trosyd Active MIX3 1:1000
*
*
**

FIG. 8

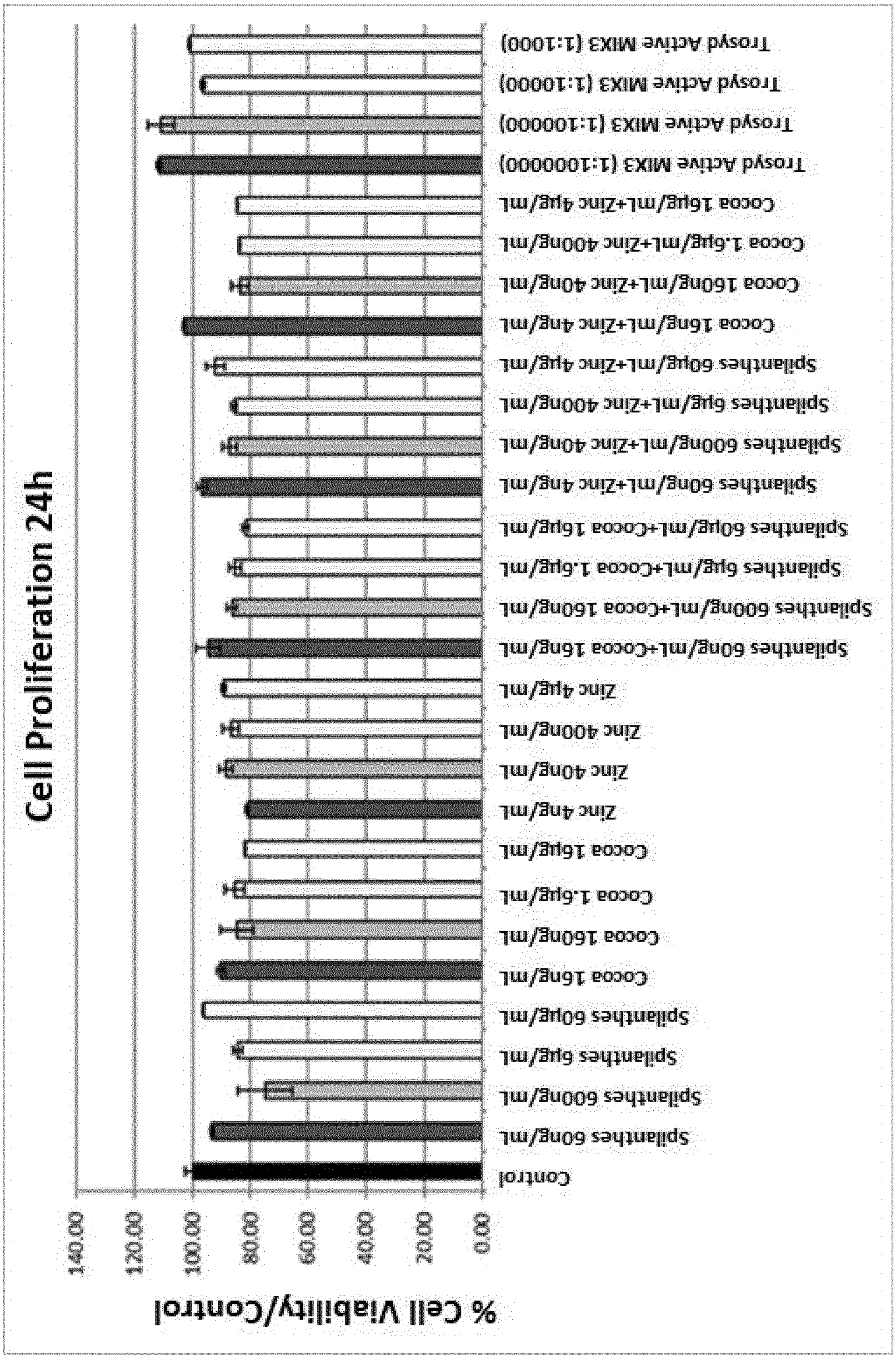
Cell Proliferation 24h
% Cell Viability/Control
0.00
20.00
40.00
60.00
80.00
100.00
120.00
140.00
Control
Spilanthes 60ng/mL
Spilanthes 600ng/mL
Spilanthes 6µg/mL
Spilanthes 60µg/mL
Cocoa 16ng/mL
Cocoa 160ng/mL
Cocoa 1.6µg/mL
Cocoa 16µg/mL
Zinc 4ng/mL
Zinc 40ng/mL
Zinc 400ng/mL
Zinc 4µg/mL
Spilanthes 60ng/mL+Cocoa 16ng/mL
Spilanthes 600ng/mL+Cocoa 160ng/mL
Spilanthes 6µg/mL+Cocoa 1.6µg/mL
Spilanthes 60µg/mL+Cocoa 16µg/mL
Spilanthes 60ng/mL+Zinc 4ng/mL
Spilanthes 600ng/mL+Zinc 40ng/mL
Spilanthes 6µg/mL+Zinc 400ng/mL
Spilanthes 60µg/mL+Zinc 4µg/mL
Cocoa 16ng/mL+Zinc 4ng/mL
Cocoa 160ng/mL+Zinc 40ng/mL
Cocoa 1.6µg/mL+Zinc 400ng/mL
Cocoa 16µg/mL+Zinc 4µg/mL
Trosyd Active MIX3 (1:1000000)
Trosyd Active MIX3 (1:100000)
Trosyd Active MIX3 (1:10000)
Trosyd Active MIX3 (1:1000)

FIG. 9

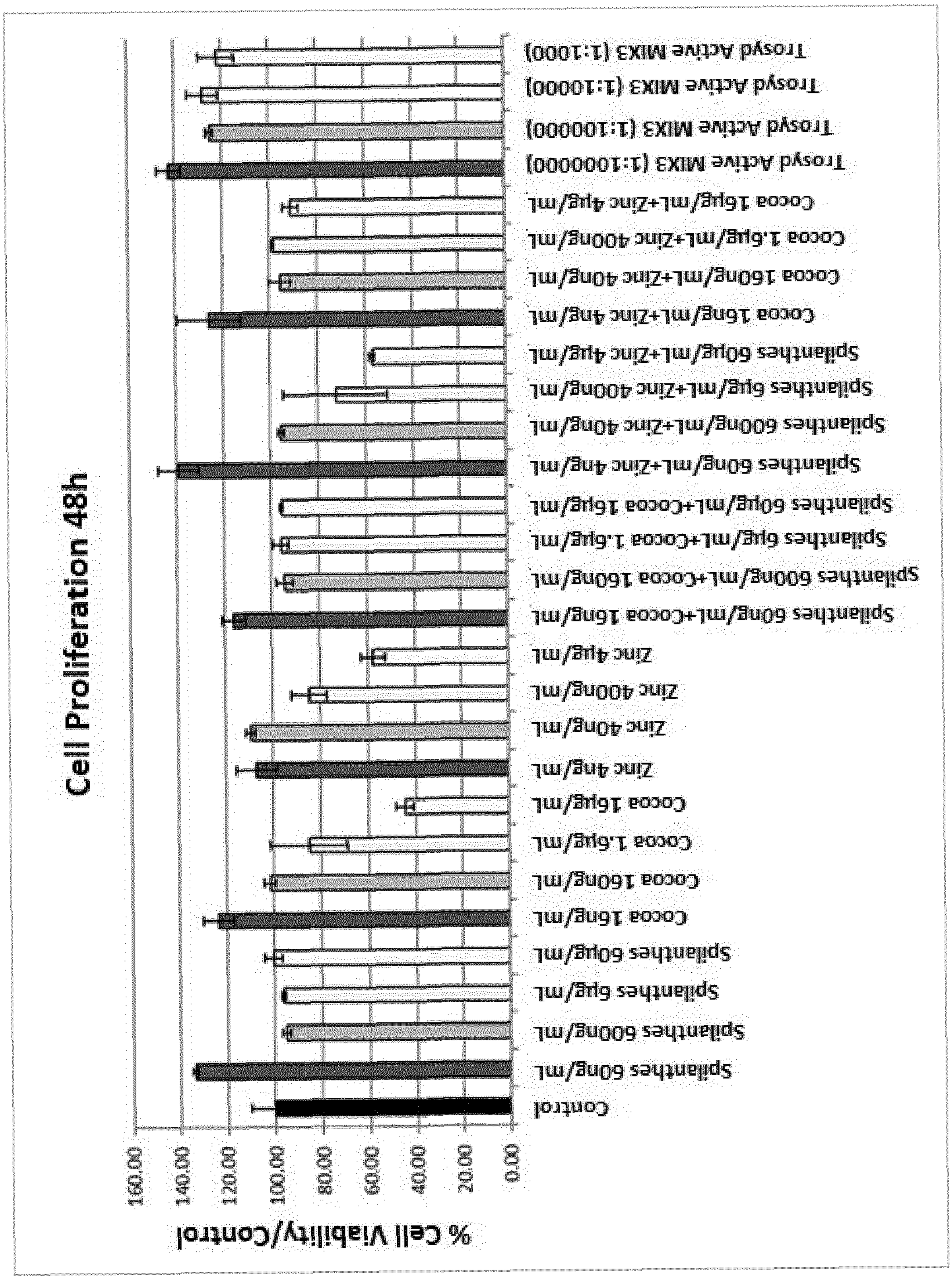
Cell Proliferation 48h
% Cell Viability/Control
160.00
140.00
120.00
100.00
80.00
60.00
40.00
20.00
0.00
Control
Spilanthes 60ng/mL
Spilanthes 600ng/mL
Spilanthes 6µg/mL
Spilanthes 60µg/mL
Cocoa 16ng/mL
Cocoa 160ng/mL
Cocoa 1.6µg/mL
Cocoa 16µg/mL
Zinc 4ng/mL
Zinc 40ng/mL
Zinc 400ng/mL
Zinc 4µg/mL
Spilanthes 60ng/mL+Cocoa 16ng/mL
Spilanthes 600ng/mL+Cocoa 160ng/mL
Spilanthes 6µg/mL+Cocoa 1.6µg/mL
Spilanthes 60µg/mL+Cocoa 16µg/mL
Spilanthes 60ng/mL+Zinc 4ng/mL
Spilanthes 600ng/mL+Zinc 40ng/mL
Spilanthes 6µg/mL+Zinc 400ng/mL
Spilanthes 60µg/mL+Zinc 4µg/mL
Cocoa 16ng/mL+Zinc 4ng/mL
Cocoa 160ng/mL+Zinc 40ng/mL
Cocoa 1.6µg/mL+Zinc 400ng/mL
Cocoa 16µg/mL+Zinc 4µg/mL
Trosyd Active MIX3 (1:1000000)
Trosyd Active MIX3 (1:100000)
Trosyd Active MIX3 (1:10000)
Trosyd Active MIX3 (1:1000)

FIG. 10

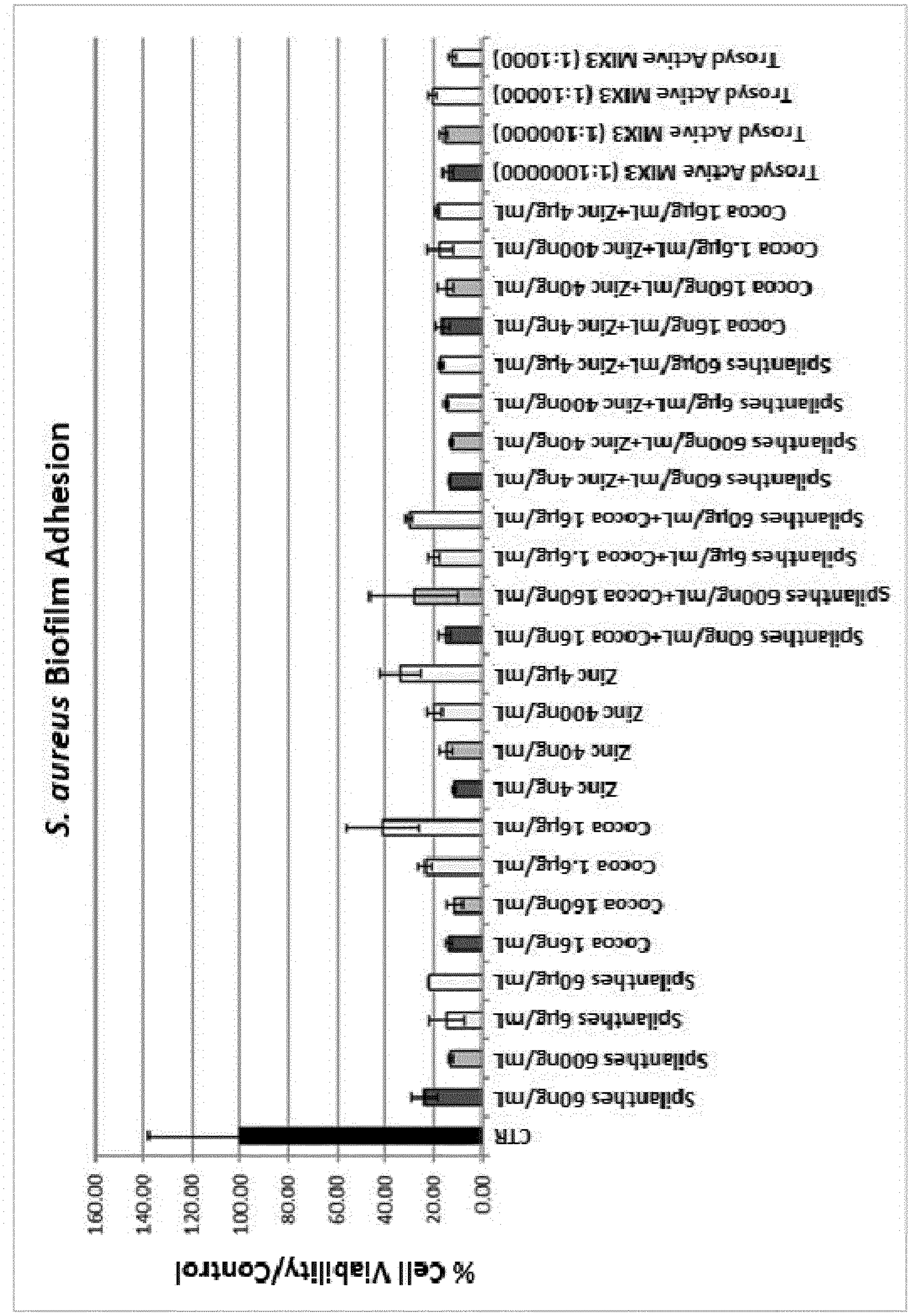
S. aureus Biofilm Adhesion
% Cell Viability/Control
160.00
140.00
120.00
100.00
80.00
60.00
40.00
20.00
0.00
CTR
Spilanthes 60ng/ml
Spilanthes 600ng/ml
Spilanthes 6μg/ml
Spilanthes 60μg/ml
Cocoa 16ng/ml
Cocoa 160ng/ml
Cocoa 1.6μg/ml
Cocoa 16μg/ml
Zinc 4ng/mL
Zinc 40ng/mL
Zinc 400ng/mL
Zinc 4μg/mL
Spilanthes 60ng/mL+Cocoa 16ng/mL
Spilanthes 600ng/mL+Cocoa 160ng/mL
Spilanthes 6μg/mL+Cocoa 1.6μg/mL
Spilanthes 60μg/mL+Cocoa 16μg/mL
Spilanthes 60ng/mL+Zinc 4ng/mL
Spilanthes 600ng/mL+Zinc 40ng/mL
Spilanthes 6μg/mL+Zinc 400ng/mL
Spilanthes 60μg/mL+Zinc 4μg/mL
Cocoa 16ng/mL+Zinc 4ng/mL
Cocoa 160ng/mL+Zinc 40ng/mL
Cocoa 1.6μg/mL+Zinc 400ng/mL
Cocoa 16μg/mL+Zinc 4μg/mL
Trosyd Active MIX3 (1:1000000)
Trosyd Active MIX3 (1:100000)
Trosyd Active MIX3 (1:10000)
Trosyd Active MIX3 (1:1000)

FIG. 11

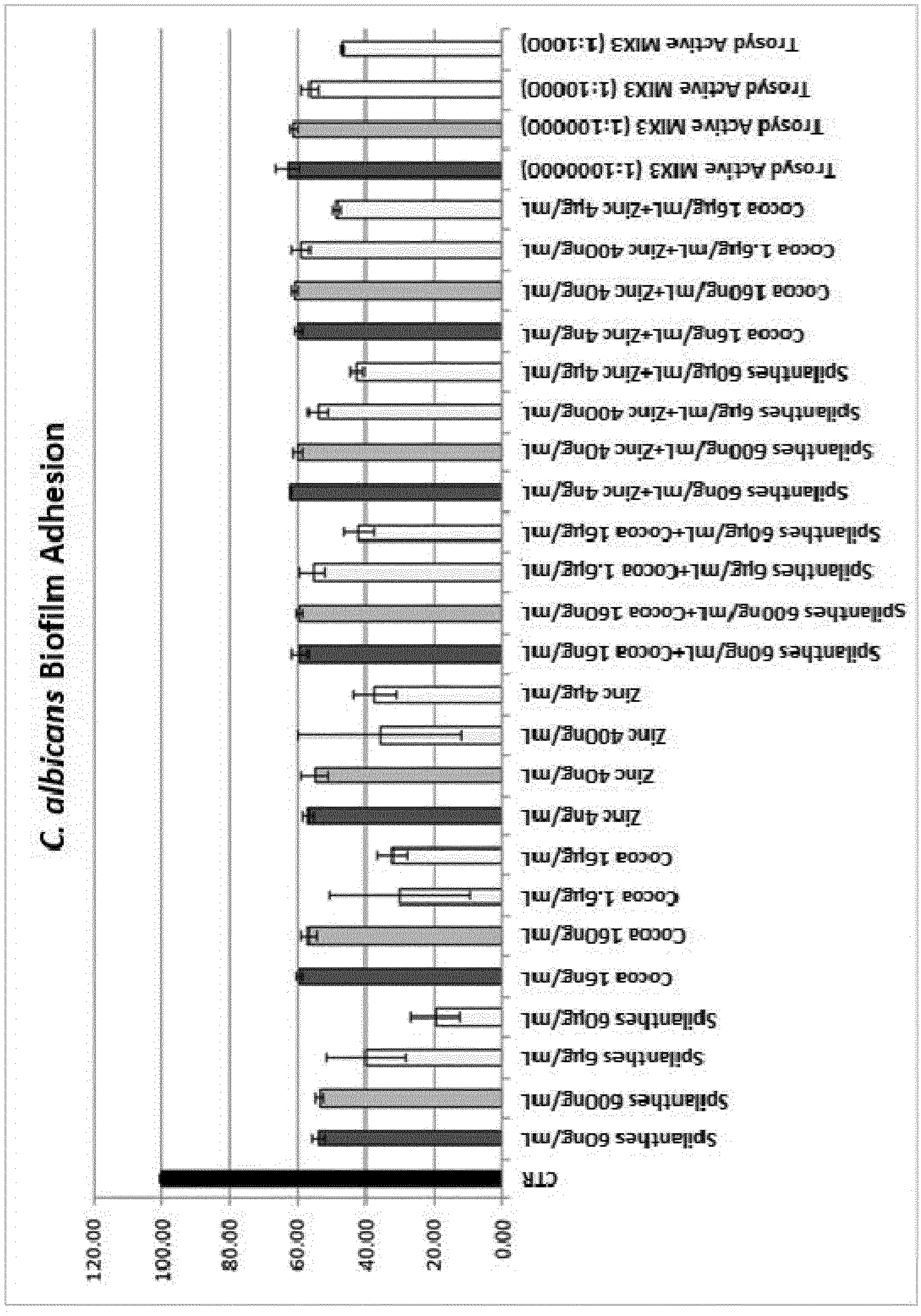
C. albicans Biofilm Adhesion
120.00
100.00
80.00
60.00
40.00
20.00
0.00
CTR
Spilanthes 60ng/mL
Spilanthes 600ng/mL
Spilanthes 6µg/mL
Spilanthes 60µg/mL
Cocoa 16ng/mL
Cocoa 160ng/mL
Cocoa 1.6µg/mL
Cocoa 16µg/mL
Zinc 4ng/mL
Zinc 40ng/mL
Zinc 400ng/mL
Zinc 4µg/mL
Spilanthes 60ng/mL+Cocoa 16ng/mL
Spilanthes 600ng/mL+Cocoa 160ng/mL
Spilanthes 6µg/mL+Cocoa 1.6µg/mL
Spilanthes 60µg/mL+Cocoa 16µg/mL
Spilanthes 60ng/mL+Zinc 4ng/mL
Spilanthes 600ng/mL+Zinc 40ng/mL
Spilanthes 6µg/mL+Zinc 400ng/mL
Spilanthes 60µg/mL+Zinc 4µg/mL
Cocoa 16ng/mL+Zinc 4ng/mL
Cocoa 160ng/mL+Zinc 40ng/mL
Cocoa 1.6µg/mL+Zinc 400ng/mL
Cocoa 16µg/mL+Zinc 4µg/mL
Trosyd Active MIX3 (1:1000000)
Trosyd Active MIX3 (1:100000)
Trosyd Active MIX3 (1:10000)
Trosyd Active MIX3 (1:1000)

FIG. 12

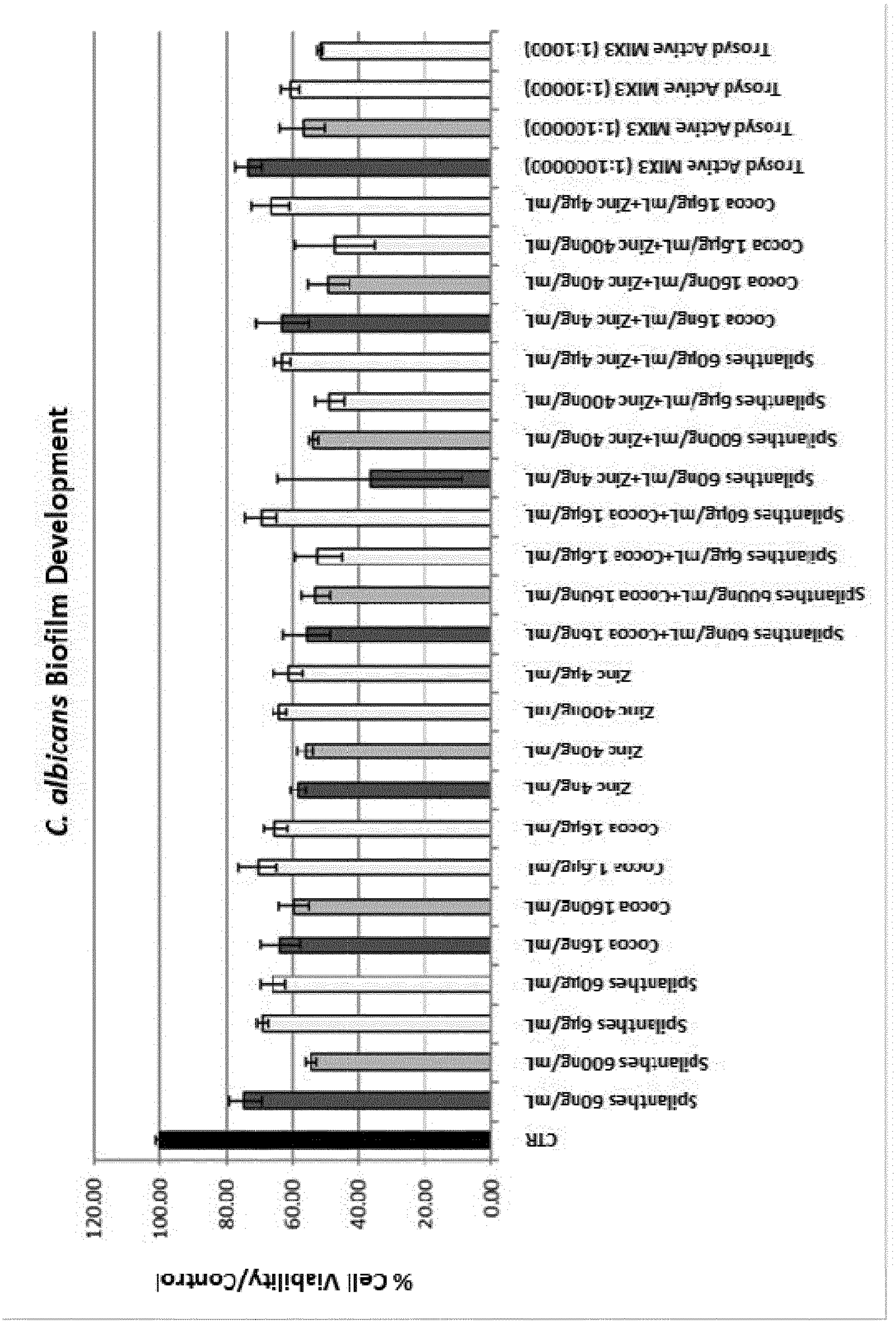
C. albicans Biofilm Development
% Cell Viability/Control
0.00
20.00
40.00
60.00
80.00
100.00
120.00
CTR
Spilanthes 60ng/mL
Spilanthes 600ng/mL
Spilanthes 6µg/mL
Spilanthes 60µg/mL
Cocoa 16ng/mL
Cocoa 160ng/mL
Cocoa 1.6µg/ml
Cocoa 16µg/mL
Zinc 4ng/mL
Zinc 40ng/mL
Zinc 400ng/mL
Zinc 4µg/mL
Spilanthes 60ng/mL+Cocoa 16ng/mL
Spilanthes 600ng/mL+Cocoa 160ng/mL
Spilanthes 6µg/mL+Cocoa 1.6µg/mL
Spilanthes 60µg/mL+Cocoa 16µg/mL
Spilanthes 60ng/mL+Zinc 4ng/mL
Spilanthes 600ng/ml+Zinc 40ng/mL
Spilanthes 6µg/mL+Zinc 400ng/mL
Spilanthes 60µg/mL+Zinc 4µg/mL
Cocoa 16ng/mL+Zinc 4ng/mL
Cocoa 160ng/mL+Zinc 40ng/mL
Cocoa 1.6µg/mL+Zinc 400ng/mL
Cocoa 16µg/mL+Zinc 4µg/mL
Trosyd Active MIX3 (1:1000000)
Trosyd Active MIX3 (1:100000)
Trosyd Active MIX3 (1:10000)
Trosyd Active MIX3 (1:1000)

FIG. 13

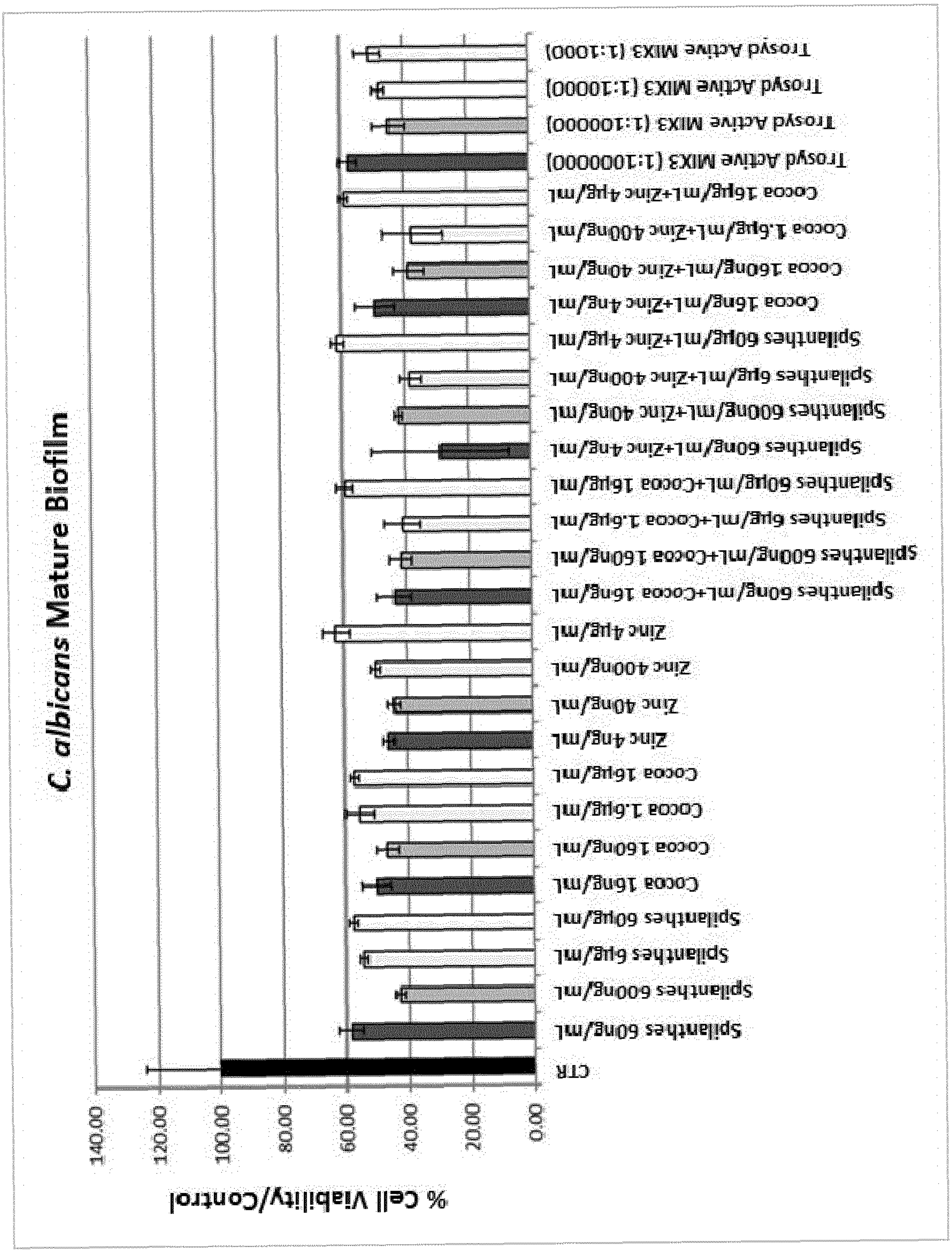
C. albicans Mature Biofilm
% Cell Viability/Control
0.00
20.00
40.00
60.00
80.00
100.00
120.00
140.00
CTR
Spilanthes 60ng/mL
Spilanthes 600ng/mL
Spilanthes 6μg/mL
Spilanthes 60μg/mL
Cocoa 16ng/mL
Cocoa 160ng/mL
Cocoa 1.6μg/mL
Cocoa 16μg/mL
Zinc 4ng/mL
Zinc 40ng/mL
Zinc 400ng/mL
Zinc 4μg/mL
Spilanthes 60ng/mL+Cocoa 16ng/mL
Spilanthes 600ng/mL+Cocoa 160ng/mL
Spilanthes 6μg/mL+Cocoa 1.6μg/mL
Spilanthes 60μg/mL+Cocoa 16μg/mL
Spilanthes 60ng/mL+Zinc 4ng/mL
Spilanthes 600ng/mL+Zinc 40ng/mL
Spilanthes 6μg/mL+Zinc 400ng/mL
Spilanthes 60μg/mL+Zinc 4μg/mL
Cocoa 16ng/mL+Zinc 4ng/mL
Cocoa 160ng/mL+Zinc 40ng/mL
Cocoa 1.6μg/mL+Zinc 400ng/mL
Cocoa 16μg/mL+Zinc 4μg/mL
Trosyd Active MIX3 (1:1000000)
Trosyd Active MIX3 (1:100000)
Trosyd Active MIX3 (1:10000)
Trosyd Active MIX3 (1:1000)

Europäisches Patentamt
European Patent Office
Office européen des brevets

# EUROPEAN SEARCH REPORT

Application Number
EP 22 18 6396

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2021/169774 A1 (KALAHASTI GEETHA [US] ET AL) 10 June 2021 (2021-06-10)<br>* paragraphs [0010], [0024], [0026], [0028], [0077], [0078]; claims 1-20 * | 1,2<br>3-10 | INV.<br>A61K31/16<br>A61K36/185<br>A61K36/28<br>A61K31/198<br>A61K31/593<br>A61K33/04<br>A61K33/30<br>A61P17/00<br>A61K8/23<br>A61K8/27<br>A61K8/44<br>A61K8/67<br>A61K8/9789<br>A61P17/06<br>A61P29/00<br>A61P31/04<br>A61P31/10 |
| X | EP 2 236 489 A1 (TAKASAGO PERFUMERY CO LTD [JP]) 6 October 2010 (2010-10-06)<br>* paragraphs [0001] - [0004], [0114] * | 1,5 | |
| Y | US 2007/041922 A1 (REINHART GALE M [US] ET AL) 22 February 2007 (2007-02-22)<br>* paragraphs [0008] - [0012], [0016]; claim 1; examples 3-5 * | 1-10 | |
| Y | US 2018/369115 A1 (ALMINANA DOMENECH NURIA [ES] ET AL) 27 December 2018 (2018-12-27)<br>* paragraphs [0160], [0165] - [0179] * | 1-10 | |
| Y | US 2018/207218 A1 (DEO NAMITA [US]) 26 July 2018 (2018-07-26)<br>* paragraphs [0051] - [0065], [0078], [0116]; claims 1-19 * | 1-10 | TECHNICAL FIELDS SEARCHED (IPC) |
| Y | WO 2019/241146 A1 (SEATTLE GUMMY COMPANY [US]) 19 December 2019 (2019-12-19)<br>* Summary;<br>page 17, paragraph 2-4 *<br>* page 25, lines 2-3 *<br>* page 26, paragraph 1 *<br>* page 27, line 4; example 21 * | 1-10 | A61K<br>A61P<br>A61Q |

-/--

The present search report has been drawn up for all claims

1

| Place of search | Date of completion of the search | Examiner |
|---|---|---|
| Munich | 29 November 2022 | Markopoulos, Eytyxia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 18 6396

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Natural Botanical Extracts", Carrubba, 26 February 2007 (2007-02-26), pages 1-247, XP055151596, Retrieved from the Internet: URL:http://www.carrubba.com/pdf/Carrubba_Botanical_Guide_r.pdf [retrieved on 2014-11-07] * item "cocoa extract"; page 121 * ----- | 1-10 | A61Q3/02 |

TECHNICAL FIELDS SEARCHED (IPC)

The present search report has been drawn up for all claims

1

| Place of search | Date of completion of the search | Examiner |
|---|---|---|
| Munich | 29 November 2022 | Markopoulos, Eytyxia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 18 6396

This annex lists the patent family members relating to the patent documents cited in the above-mentioned European search report. The members are as contained in the European Patent Office EDP file on
The European Patent Office is in no way liable for these particulars which are merely given for the purpose of information.

29-11-2022

| Patent document cited in search report | Publication date | Patent family member(s) | Publication date |
|---|---|---|---|
| US 2021169774 A1 | 10-06-2021 | CN 115087427 A | 20-09-2022 |
| | | EP 4072513 A1 | 19-10-2022 |
| | | US 2021169774 A1 | 10-06-2021 |
| | | US 2022323341 A1 | 13-10-2022 |
| | | WO 2021119664 A1 | 17-06-2021 |
| EP 2236489 A1 | 06-10-2010 | CN 101910116 A | 08-12-2010 |
| | | EP 2236489 A1 | 06-10-2010 |
| | | JP 5401708 B2 | 29-01-2014 |
| | | JP WO2009091040 A1 | 26-05-2011 |
| | | US 2010184863 A1 | 22-07-2010 |
| | | US 2011105773 A1 | 05-05-2011 |
| | | US 2014227200 A1 | 14-08-2014 |
| | | WO 2009091040 A1 | 23-07-2009 |
| US 2007041922 A1 | 22-02-2007 | NONE | |
| US 2018369115 A1 | 27-12-2018 | AU 2016366220 A1 | 28-06-2018 |
| | | BR 112018011643 A2 | 04-12-2018 |
| | | CN 108495646 A | 04-09-2018 |
| | | EP 3386528 A1 | 17-10-2018 |
| | | JP 7010823 B2 | 26-01-2022 |
| | | JP 2019502677 A | 31-01-2019 |
| | | KR 20180094008 A | 22-08-2018 |
| | | US 2018369115 A1 | 27-12-2018 |
| | | WO 2017100421 A1 | 15-06-2017 |
| US 2018207218 A1 | 26-07-2018 | US 2016074460 A1 | 17-03-2016 |
| | | US 2018207218 A1 | 26-07-2018 |
| | | US 2019388490 A1 | 26-12-2019 |
| WO 2019241146 A1 | 19-12-2019 | CN 112312915 A | 02-02-2021 |
| | | EP 3801531 A1 | 14-04-2021 |
| | | US 2021228483 A1 | 29-07-2021 |
| | | WO 2019241146 A1 | 19-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BOUKAMP P. ; DZARLIEVA-PETRUSSEVSKA R.T. ; BREITKREUTZ D., H J. ; MARKHAM A. ; FUSENIG N.E.** *Normal keratinization in a spontaneously immortalized human keratinocyte cell line.J.,* 1988, vol. 106, 761-771
- **SCHURER N. ; KOHNE A. ; SCHLIEP V. ; BARLAG K. ; GOERZ G.** Lipid composition and synthesis of HaCaT cells, animmortalized keratinocyte line, in comparison with normal human adult keratinocytes. *Exp. Dermatol.,* 1993, vol. 2, 179-185
- **LEHMAN R.T. ; MODALI R. ; BOUKAMP P. ; STANEK J. ; BENNETT W WELSH J.A. ; METCALF R.A. ; STAMPFER M.R. ; FUSENIG N.E. ; ROG HARRIS C.C.** *mutations in human immortalized epithelial ○ Carcinogenesis,* 1993, vol. 839, 53